(19) Europäisches Patentamt
European Patent Office
Office européen des brevets

(11) EP 1 874 761 B1

(12) FASCICULE DE BREVET EUROPEEN

(45) Date de publication et mention de la délivrance du brevet:
**18.08.2010 Bulletin 2010/33**

(21) Numéro de dépôt: **06755440.2**

(22) Date de dépôt: **21.04.2006**

(51) Int Cl.:
***C07D 403/04*** (2006.01) ***C07D 253/06*** (2006.01)
***C07D 453/02*** (2006.01) ***C07D 405/04*** (2006.01)
***C07D 409/04*** (2006.01)

(86) Numéro de dépôt international:
**PCT/FR2006/000887**

(87) Numéro de publication internationale:
**WO 2006/111662 (26.10.2006 Gazette 2006/43)**

# (54) DERIVES DE 4H-1,2,4-TRIAZIN-5-0NES, LEUR PREPARATION ET LEUR UTILISATION COMME DES RECEPTEURS A L'ACETYLCHOLINE DE TYPE NICOTINIQUES ALPHA7

4H-1,2,4-TRIAZIN-5-ON-DERIVATE, IHRE HERSTELLUNG UND IHRE VERWENDUNG ALS ALPHA-7-NIKOTIN-ACETYLCHOLIN-REZEPTOREN

4H-1,2,4-TRIAZIN-5-ONE DERIVATIVES, THEIR PREPARATION AND THEIR USE AS ALPHA 7 NICOTINIC ACETYLCHOLINE RECEPTORS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **22.04.2005 FR 0504042**

(43) Date de publication de la demande:
**09.01.2008 Bulletin 2008/02**

(73) Titulaire: **Aventis Pharma S.A.**
**92160 Antony (FR)**

(72) Inventeurs:
- **BENEDETTI, Yannick**
  **F-93110 Rosny-sous-Bois (FR)**
- **BOHME, Andrees**
  **F-75020 Paris (FR)**
- **GENEVOIS-BORELLA, Arielle**
  **F-94320 Thiais (FR)**
- **TOUYER, Gaetan**
  **F-77500 Chelles (FR)**
- **ZHANG, Jidong**
  **F-75013 Paris (FR)**

(74) Mandataire: **Gaslonde, Aude**
**Département Brevets**
**20 Avenue Raymond Aron**
**92160 Antony (FR)**

(56) Documents cités:
**WO-A-03/029252**

- **PATENT ABSTRACTS OF JAPAN vol. 2002, no. 05, 3 mai 2002 (2002-05-03) -& JP 2002 030084 A (MITSUBISHI PHARMA CORP), 29 janvier 2002 (2002-01-29)**
- **RIED, WALTER ET AL: "Nucleophilic reactions on chloro-N-(2-oxoacyl)formamidines" LIEBIGS ANNALEN DER CHEMIE , (2), 141-8 CODEN: LACHDL; ISSN: 0170-2041, 1988, XP001149027**
- **LAVERGNE, JEAN PIERRE ET AL: "Research in the azabenzodiazepine series. IV. 1-(3'- Triazinyl) ethylenediamines, synthetic intermediates for 7,8,9,10-tetrahydro-(2H)(6H)-as-triazino[2 ,3-c]-1,3,5-triazepines" BULLETIN DE LA SOCIETE CHIMIQUE DE FRANCE , (11-12, PT. 2), 1827-8 CODEN: BSCFAS; ISSN: 0037-8968, 1976, XP009056964**
- **LALEZARI, IRADJ: "1,2,4-Triazines. VIII. The synthesis of 1,2,4-triazino[2,3- e]pyrazolo[1,5-a]-1,3,5-triazines and 1,2,4-triazino[4,3-e]pyrazolo [1,5-a]- 1,3,5-triazines" JOURNAL OF HETEROCYCLIC CHEMISTRY , 13(6), 1249-51 CODEN: JHTCAD; ISSN: 0022-152X, 1976, XP002353864**
- **BURCH, HOMER A.: "Nitrofuryl heterocycles. X. Analogs of 6-(5-nitro-2-furyl)-as-triazine- 3,5(2H, 4H)-dione" JOURNAL OF MEDICINAL CHEMISTRY , 13(2), 288-91 CODEN: JMCMAR; ISSN: 0022-2623, 1970, XP001119262**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 874 761 B1

## Description

L'invention se rapporte à des dérivés de triazines, à leur préparation et à leur application en thérapeutique.

Les composés de l'invention sont de nouveaux ligands des récepteurs à l'acétylcholine de type nicotiniques. Ces composés sont caractérisés plus particulièrement en ce qu'ils sont des ligands des récepteurs nicotiniques de type $\alpha 7$.

La présente invention a pour objet les composés répondant à la formule (I)

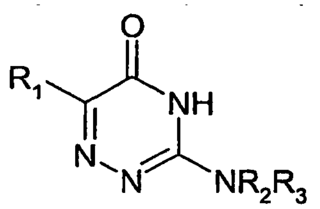

(I)

dans laquelle

$R_1$ représente un groupe hétéroaryle ou aryle, ledits groupes hétéroaryle ou aryle étant éventuellement substitués par un ou plusieurs groupes choisis parmi les atomes d'halogène et les groupes $(C_1\text{-}C_4)$alkyle, $(C_1\text{-}C_4)$alcoxy, $(C_3\text{-}C_7)$cycloalkyle, aryle, hydroxy, cyano, $-NH_2$, $-NO_2$;
$R_2$ représente un atome d'hydrogène ou un groupe $(C_1\text{-}C_4)$alkyle;
$R_3$ représente

- un groupe $-(CH_2)n-NR_4R_5$ dans lequel

  n est égal à 2, 3 ou 4 et
  $R_4$ et $R_5$ représentent, indépendamment l'un de l'autre, un groupe $(C_1\text{-}C_4)$alkyle, ou $(C_3\text{-}C_7)$cycloalkyle, ou bien $R_4$ et $R_5$ forment ensemble, avec l'atome d'azote qui les porte, un groupe $(C_3\text{-}C_9)$hétérocycloalkyle; ou

- un groupe $-(CH_2)mR_6$ dans lequel

  m est égal à 0, 1, 2, 3 ou 4 et
  $R_6$ représente un groupe un groupe $(C_3\text{-}C_9)$hétérocycloalkyle comportant au moins un atome d'azote et lié au noyau triazine par un atome de carbone, le groupe $(C_3\text{-}C_9)$hétérocycloalkyle étant éventuellement substitué par un ou plusieurs groupes $(C_1\text{-}C_4)$alkyle,

ou bien $R_2$ et $R_3$ forment ensemble, avec l'atome d'azote qui les porte, un $(C_5\text{-}C_9)$hétérocycloalkyle comprenant 2 atomes d'azote ;
avec la condition que quand $R_1$ représente un phényle alors $R_4$ et $R_5$ ne représentent pas simultanément un méthyle.

Le composé 3-[[2-(diméthylarnino)éthyl]amino]-6-phényl-1,2,4-triazin-5(2H)-one (Chemical Library ; RN : 451523-47-0) a été décrit sans aucune propriété pharmacologique associée.

Il est exclu de la formule générale (I) de l'invention.

Le document JP2002030084 divulgue des dérivés de 1-azabicycloalcane en tant qu'agonistes des récepteurs nicotiniques de typa alpha 7.

Le document WO03/029252 A1 décrit des dérivés d'hétéroarylcarboxamides substitués par des azabicycles ayant une activité sur les récepteurs de type alpha 7.

Les documents Ried W. and al., Liebigs annalen der chemie, (2). 141-8, 1988 ; Lavergne, J.-P. and al., Bulletin de la Société Chimique de France, (11-12, PT. 2), 1827-8, 1976; Lalezari, I., Journal of Heterocyclic Chemistry, 13(6), 1249-51, 1976 décrivent la synthèse de dérivés de triazines sans aucune propriété pharmacologique associée.

Le document Burch, H., Journal of Medicinal Chemistry, 13(2), 288-91, 1970 décrit la synthèse de dérivés de triazines testés pour leur activité sur le système urinaire.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :

- $(C_t-C_z)$ où t et z peuvent prendre les valeurs de 1 à 9 représente une chaîne carbonée pouvant avoir de 1 à 9 atomes de carbone ;
- un atome d'halogène : un fluor, un chlore, un brome ou un iode ;
- un groupe alkyle : un groupe aliphatique saturé linéaire ou ramifié. A titre d'exemples, on peut citer les groupes méthyle, éthyle, propyle, isopropyle, butyle, isobutyle, tertbutyle, pentyle, etc ;
- un groupe alcoxy : un radical -O-alkyle où le groupe alkyle est tel que précédemment défini ;
- un groupe cycloalkyle : un groupe alkyle cyclique. A titre d'exemples, on peut citer les groupes cyclopropyle, cyclobutyle, cyclopentyle, cyclohexyle, etc ;
- un groupe hétérocycloalkyle : un hétérocycle saturé comprenant de 1 à 2 hétéroatomes, tels que l'azote, l'oxygène ou le soufre, et étant éventuellement ponté, c'est-à-dire comportant éventuellement une liaison carbonée de 1 à 3 atomes de carbone entre 2 atomes du cycle. A titre d'exemples de groupes hétérocycloalkyle, on peut citer les groupes pyrrolidinyle, pipéridinyle, perhydro-azépinyle, perhydro-1,4-oxazépinyle, perhydro-1,4-thiazépinyle, perhydro-1,4-diazépinyle, 7-aza-bicyclo[2.2.1]heptanyle, 1-aza-bicyclo[2.2.1]heptanyle , 1-aza-bicyclo[2.2.2]octanyle, 2-aza-bicyclo[2.2.2]octanyle, 1-aza-bicyclo[3.2.1]octanyle, 8-aza-bicyclo[3.2.1]octanyle, 3-aza-bicyclo[3.2.1]octanyle, 1-aza-bicyclo[3.2.2]nonanyle, 3-aza-bicyclo[3.2.2]nonanyle, 1-aza-bicyclo[3.3.1]nonanyle, 3-aza-bicyclo[3.3.1]nonanyle, 9-aza-bicyclo[3.3.1]nonanyle, 1,4-diaza-bicyclo[3.2.2]nonanyle ;
- un groupe aryle : un groupe aromatique cyclique comprenant de 6 à 10 atomes de carbone. A titre d'exemples de groupes aryles, on peut citer les groupes phényle, naphtyle, indènyle ;
- un groupe hétéroaryle : un groupe aromatique cyclique comprenant de 1 à 9 atomes de carbone et comprenant de 1 à 3 hétéroatomes, tels que l'azote, l'oxygène ou le soufre. A titre d'exemples de groupes hétéroaryles, on peut citer les groupes furanyle, thiènyle, pyrrolyle, oxazolyle, thiazolyle, imidazolyle, pyrazolyle, isoxazolyle, isothiazolyle, isoxadiazolyle, isothiadiazolyle, oxadiazolyle, triazolyle, tétrazolyle, thiadiazolyle, pyridinyle, pyridazinyle, pyrimidinyle, pyrazinyle, triazinyle, indolyle, isoindolyle, benzofuranyle, benzothiènyle, indazolyle, benzimidazolyle, benzothiazolyle, quinolinyle, isoquinolinyle, azaindolyle.

Parmi les composés de formule (I) objets de l'invention, un premier sous-groupe de composés est constitué par les composés pour lesquels :

$R_1$ représente un groupe hétéroaryle, plus particulièrement un furanyle, thiènyle, pyrrolyle, oxazolyle, thiazolyle, imidazolyle, pyrazolyle, isoxazolyle, isothiazolyle, isoxadiazolyle, isothiadiazolyle, oxadiazolyle, triazolyle, tétrazolyle, thiadiazolyle, pyridinyle, pyridazinyle, pyrimidinyle, pyrazinyle, triazinyle, indolyle, isoindolyle, benzofuranyle, benzothiènyle, indazolyle, benzimidazolyle, benzothiazolyle, quinolinyle, isoquinolinyle, azaindolyle, ledit groupe hétéroaryle étant éventuellement substitué par un ou plusieurs groupes, plus particulièrement par un ou deux groupes, choisis parmi les atomes d'halogène et les groupes $(C_1-C_4)$alkyle, $(C_1-C_4)$alcoxy, $(C_3-C_7)$cycloalkyle, aryle, hydroxy, cyano, $-NH_2$, $-NO_2$.

Parmi les composés de formule (I) objets de l'invention, un second sous-groupe de composés est constitué par les composés pour lesquels :

$R_1$ représente un groupe hétéroaryle, plus particulièrement un furanyle, thiènyle, pyrrolyle, pyrazolyle, triazolyle, imidazolyle, indolyle, indazolyle, benzothiènyle, azaindolyle ou aryle, plus particulièrement phényle ou naphtyle, ledits groupes hétéroaryle ou aryle étant éventuellement substitués par un ou plusieurs groupes, plus particulièrement par un ou deux groupes, choisis parmi les atomes d'halogène, plus particulièrement, de fluor, et les groupes $(C_1-C_4)$alkyle, plus particulièrement méthyle, $(C_1-C_4)$alcoxy, plus particulièrement méthoxy, aryle, plus particulièrement phényle.

Parmi les composés de formule (I) objets de l'invention, un troisième sous-groupe de composés est constitué par les composés pour lesquels :

$R_1$ représente un groupe hétéroaryle, plus particulièrement un furanyle, thiènyle, pyrrolyle, pyrazolyle, triazolyle,

imidazolyle, indolyle, indazolyle, benzothiènyle, azaindolyle, ledit groupe hétéroaryle étant éventuellement substitué par un ou plusieurs groupes, plus particulièrement par un ou deux groupes, choisis parmi les atomes d'halogène, plus particulièrement, de fluor, et les groupes $(C_1-C_4)$alkyle, plus particulièrement méthyle, $(C_1-C_4)$alcoxy, plus particulièrement méthoxy, aryle, plus particulièrement phényle.

Parmi les composés de formule (I) objets de l'invention, un quatrième sous-groupe de composés est constitué par les composés pour lesquels :

$R_2$ représente un atome d'hydrogène.

Parmi les composés de formule (I) objets de l'invention, un cinquième sous-groupe de composés est constitué par les composés pour lesquels :

$R_3$ représente

- un groupe $-(CH_2)n-NR_4R_5$ dans lequel

  n est égal à 2, 3 ou 4, et
  $R_4$ et $R_5$ représentent, indépendamment l'un de l'autre, un groupe $(C_1-C_4)$alkyle, plus particulièrement méthyle ou éthyle, ou bien $R_4$ et $R_5$ forment ensemble, avec l'atome d'azote qui les porte, un groupe $(C_3-C_7)$hétérocycloalkyle, plus particulièrement pyrrolidinyle ou pipéridinyle ; ou

- un groupe $-(CH_2)mR_6$ dans lequel

  m est égal à 0, 1, 2, 3 ou 4, plus particulièrement 0 ou 2, et
  $R_6$ représente un groupe $(C_3-C_7)$hétérocycloalkyle comportant au moins un atome d'azote et lié au noyau triazine par un atome de carbone, plus particulièrement pyrrolidinyle ou quinuclidinyle, le groupe $(C_3-C_7)$hétérocycloalkyle étant éventuellement substitué par un ou plusieurs groupes $(C_1-C_4)$alkyle, plus particulièrement méthyle ;

avec la condition que quand $R_1$ représente un phényle alors $R_4$ et $R_5$ ne représentent pas simultanément un méthyle.
Parmi les composés de formule (I) objets de l'invention, un sixième sous-groupe de composés est constitué par les composés pour lesquels à la fois
$R_1$ est tel que défini dans le premier, le second ou le troisième sous-groupe ci-dessus ;
$R_2$ est tel que défini dans le quatrième sous-groupe ci-dessus ;
$R_3$ est tel que défini dans le cinquième sous-groupe ci-dessus.
Parmi les composés de formule (I) objets de l'invention, un septième sous-groupe de composés est constitué par les composés pour lesquels :

$R_1$ représente un groupe indolyle, ledit groupe indolyle étant éventuellement substitué par un ou plusieurs groupes, plus particulièrement par un ou deux groupes, choisis parmi les atomes d'halogène, plus particulièrement, de fluor et de chlore, et les groupes $(C_1-C_4)$alkyle, plus particulièrement méthyle, $(C_1-C_4)$alcoxy, plus particulièrement méthoxy, $(C_3-C_7)$cycloalkyle, plus particulièrement cyclohexyle, aryle, plus particulièrement phényle, $-NH_2$, $-NO_2$; et/ou
$R_2$ représente un atome d'hydrogène ; et/ou
$R_3$ représente

- un groupe $-(CH_2)n-NR_4R_5$ dans lequel

  n est égal à 2, 3 ou 4, et
  $R_4$ et $R_5$ représentent, indépendamment l'un de l'autre, un groupe $(C_1-C_4)$alkyle, plus particulièrement méthyle ou éthyle, ou bien $R_4$ et $R_5$ forment ensemble, avec l'atome d'azote qui les porte, un groupe $(C_3-C_7)$hétérocydoalkyle, plus particulièrement pyrrolidinyle ou pipéridinyle ; ou

- un groupe $-(CH_2)mR_6$ dans lequel

  m est égal à 0, 1, 2, 3 ou 4, plus particulièrement 0 ou 2, et
  $R_6$ représente un groupe $(C_3-C_7)$hétérocycloalkyle, plus particulièrement pyrrolidinyle ou quinuclidinyle, le groupe $(C_3-C_7)$hétérocycloalkyle étant éventuellement substitué par un ou plusieurs groupes $(C_1-C_4)$alkyle,

plus particulièrement méthyle.

Parmi les composés de formule (I) objets de l'invention, on peut notamment citer les composés suivants :

- 6-(1-méthyl-1*H*-indol-3-yl)-3-(3-pipéfidin-1-yl-propylamino)-4*H*-1,2,4-triazin-5-one
- 3-(3-diéthylamino-propylamino)-6-(1-méthyl-1*H*-indol-3-yl)-4*H*-1,2,4-triazin-5-one
- 3-(3-diéthylamino-propylamino)-6-phényl-4*H*-1,2,4-triazin-5-one
- 3-(2-diéthylamino-éthylamino)-6-(1-méthyl-1-*H*-indol-3-yl)-4*H*-1,2,4-triazin-5-one
- 3-(3-diméthylamino-propylamino)-6-(1-méthyl-1*H*-indoi-3-yl)-4*H*-1,2,4-triazin-5-one
- 6-(1-méthyl-1*H*-indol-3-yl)-3-(3-pyrrolidin-1-yl-propylamino)-4*H*-1,2,4-triazin-5-one
- 6-(1-mëthyl-1*H*-indol-3-yl)-3-[2-(1-méthyl-pyrrolidin-2-yl)-éthylamino]-4*H*-1,2,4-triazin-5-one
- 6-(1*H*-indol-3-yl)-3-(3-pyrrolidin-1-yl-propylamino)-4*H*-1,2,4-triazin-5-one
- 6-(1*H*-indol-3-yl)-3-(3-pipéridin-1-yl-propylamino)-4*H*-1,2,4-triazin-5-one
- 3-[(*S*)-(1-aza-bicyclo[2.2.2]oct-3-yl)amino]-6-(1*H*-indol-3-yl)-4*H*-1-,2,4.triazin-5-one
- 3-[(*R*)-(1-aza-bicyclo[2.2.2]oct-3-yl)amino]-6-(1*H*-indol-3-yl)-4*H*-1,2,4-triazin-5-one)
- 6-(furan-3-yl)-3-(3-diéthylaminopropylamino)-4*H*-1,2,4-triazin-5-one)
- 6-(napht-1-yl-)-3-(3-diéthylaminopropylamino)-4*H*-1,2,4-triazin-5-one
- 6-(1-méthyl-5-méthoxy-indol-3-yl)-3-(3-diéthylaminopropylamino)-4*H*-1,2,4-triazin-5-one
- 6-(1-phényl-indol-3-yl)-3-(3-diéthylaminopropylamino)-4*H*-1,2,4-triazin-5-one
- 6-(indol-3-yl)-3-(3-diéthylaminopropylamino)-4*H*-1,2,4-triazin-5-one
- 6-(benzothiophen-3-yl)-3-(3-diéthylaminopropylamino)-4*H*-1,2,4-triazin-5-one
- 6-(4-méthoxyphényl)-3-(3-diéthylaminopropylamino)-4*H*-1,2,4-triazin-5-one
- 6-(1-méthyl-5.fluoro-indol-3-yl).3-(3-diéthylaminopropylamino)-4*H*-1,2,4-triazin-5-one
- 6-(1-méthyl-6-fluoro-indol-3-yl)-3-(3-diéthylaminopropylamino)-4*H*-1,2,4-triazin-5-one
- 6-(3-méthoxyphenyl)-3-(3-diéthylaminopropylamino)-4*H*-1,2,4-triazin-5-one
- 6-(4-(4-méthylphényl))-3-(3-diéthylaminopropylamino)-4*H*-1,2,4-triazin-5-one
- 6-(1-méthyl-4-fluor-indol-3-yl)-3-(3-diéthylaminopropylamino)-4*H*-1,2,4-triazin-5-one.

Conformément à l'invention, on peut préparer les composés de formule générale (I) selon les procédés illustrés par les schémas qui suivent.

Une première méthode de préparation des composés de formule générale (I) est illustrée dans le schéma 1.

Selon cette méthode, le composé de formule générale (II), dans laquelle $R_1$ est tel que défini dans la formule générale (I), est condensé à chaud avec du thiosemicarbazide en présence d'eau et en milieu acide concentré, pour obtenir le composé de formule générale (III).

Le composé de formule générale (III) est ensuite cyclisé à chaud et en milieu basique, par exemple en présence d'hydroxyde de sodium, pour former le composé de formule générale (IV).

Le composé de formule générale (V) est ensuite obtenu par méthylation du composé de formule générale (IV) en présence de iodure de méthyle et d'une base forte telle que l'hydrure de sodium dans un solvant tel que le diméthylformamide (DMF).

Enfin, le composé de formule générale (I) est obtenu par réaction du composé de formule générale (V) avec une amine de formule générale $HNR_2R_3$ (VI), dans laquelle $R_2$ et $R_3$ sont tels que définis dans la formule générale (I).

Schéma 1

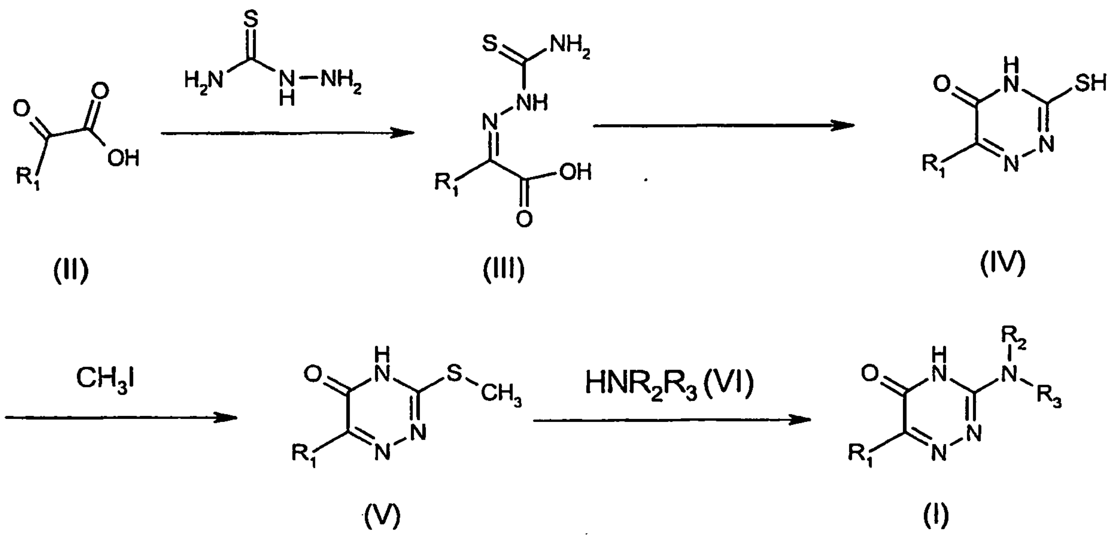


(II) (III) (IV)

(V) (I)

Une seconde méthode de préparation des composés de formule générale (I) est illustrée dans le schéma 2.

Selon cette méthode, le thiosemicarbazide est d'abord méthylé en présence d'iodure de méthyle dans un solvant tel que l'éthanol. Le produit méthylé résultant est ensuite mis en présence d'une amine de formule générale $HNR_2R_3$ (VI) , dans laquelle $R_2$ et $R_3$ sont tels que définis dans la formule générale (I), dans un solvant tel que l'éthanol, pour former le composé de formule générale (VII).

Enfin, le composé de formule générale (I) est obtenu par réaction du composé de formule générale (VII) avec le composé de formule générale (II) , dans laquelle $R_1$ est tel que défini dans la formule générale (I), dans l'eau, en chauffant par exemple en présence de micro-ondes à une température de l'ordre de 140°C.

Schéma 2

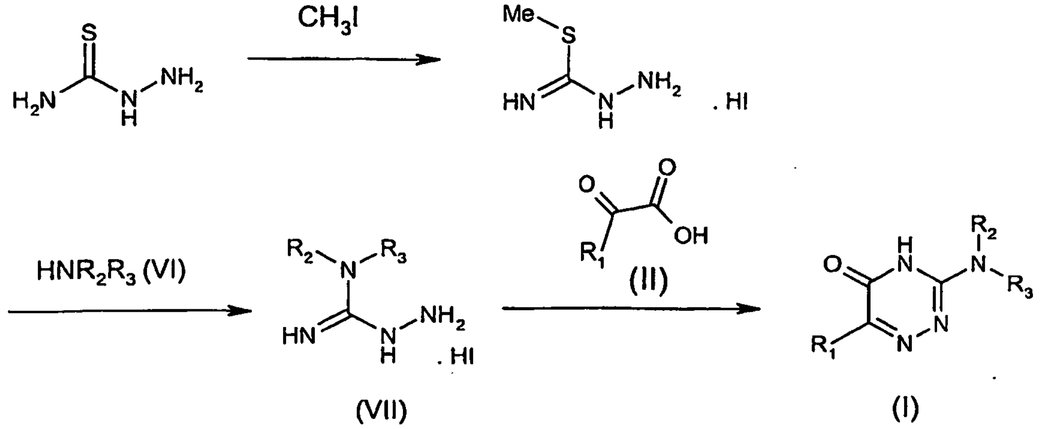


(VII) (I)

Les composés de formule générale (II) sont commerciaux, connus dans la littérature ou peuvent être préparés par des méthodes connues de l'homme de l'art. Les composés de formule générale (II) ou les esters correspondants peuvent être synthétisés par exemple par application ou adaptation des méthodes décrites dans Tetrahedron Lett., 1994, 35 (19), 3013-3016, Tetrahedron Lett., 1998, 39 (52), 9629-9630, J. Org. Chem., 2002, 67 (17), 6226-6227, Synthesis, 1998 (9), 1241-1242, J. Heterocycl. Chem., 1997, 34 (2), 441-444, J. Heterocycl. Chem., 1997, 34 (3), 789-795, Tetrahedron, 2003, 59 (7), 1083-1094, Bioorg. Med. Chem. Lett., 2002, 12 (7), 1117-1120, Heterocycles, 1998, 49 (1), 459-464, J. Org. Chem., 1981, 46 (1), 211-213, Tetrahedron, 1196, 52 (42), 13513-13520, Tetrahedron, 1999, 55 (37), 11343-11364, J. Org. Chem., 1987, 52 (26), 5733-5740, Adv. Synth. Catal., 2001, 343 (3), 289-298 ou Tetrahedron Lett., 1983, 24 (33), 3455-3456. La transformation éventuelle de l'ester d'un composé de formule générale (II) en un composé de formule générale (II) s'effectue selon les méthodes classiques connues de l'homme de l'art.

Les composés de formule générale (VI) sont commerciaux, connus dans la littérature ou peuvent être préparés par des méthodes connues de l'homme de l'art. Les composés de formule générale (VI) peuvent être synthétisés par exemple par application ou adaptation des méthodes décrites dans WO2003018585A1, WO2002085901A1, WO2003029252A1, Synth. Comm., 2002, 32 (13), 1985-1995, WO2002068420AI, EP115933A2, J. Med. Chem., 1993, 36 (6), 683-689, Tetrahedron, 1992, 49 (2), 451-468, Hev. Chim. Acta, 1955, 38 (3), 559-570, Hev. Chim. Acta 1959, 42 (1), 67-72, J. Med. Chem., 1993, 36 (16), 2311-20, WO2003070728A2, ou J. Org. Chem., 1996, 61 (11), 3766-3772.

Dans les schémas 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les exemples suivants décrivent la préparation de certains composés conformes à l'invention. Les numéros des composés exemplifiés renvoient à ceux donnés dans le tableau ci-après, qui illustre les structures chimiques et les propriétés physiques de quelques composés selon l'invention.

## Exemple 1

6-(1-méthyl-1*H*-indol-3-yl)-3-(3-pipéridin-1-yl-propylamino)-4*H*-1,2,4-triazin-5-one (composé n˚2)

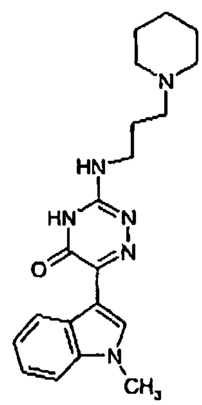

1.1 3-mercapto-6-(1-méthyl-1*H*-indol-3-yl)-4*H*-1,2,4-triazin-5-one

Une suspension de 15 g d'acide (1-méthyl-1*H*-indol-3-yl)-oxo-acétique et de 7,8 g de thiosemicarbazide dans 150 $cm^3$ d'eau est additionnée de 2,25 $cm^3$ d'acide chlorhydrique 12 N et le mélange est chauffé sous agitation 2,5 h à 100˚C. Le milieu réactionnel est refroidi dans un bain d'eau glacée et le solide en suspension est filtré, rincé à l'eau et séché à l'air. Après avoir repris le solide jaune obtenu (20,5 g) par 150 $cm^3$ d'eau et ajusté le pH de la solution à un pH voisin de 11 à l'aide d'hydroxyde de sodium 1 N, on chauffe le mélange sous agitation 4 h au reflux et on maintient l'agitation encore 15 h à une température voisine de 20˚C. Le milieu réactionnel, refroidi dans un bain de glace, est amené à un pH voisin de 4 par addition d'acide acétique. Le précipité résultant est collecté par filtration, rincé avec de l'eau et séché à l'air pendant 3 jours pour donner 18,9 g de 3-mercapto-6-(1-méthyl-1*H*-indol-3-yl)-4*H*-1,2,4-triazin-5-one sous la forme d'un solide jaune.

1.2 6-(1-méthyl-1*H*-indol-3-yl)-3-méthylsulfanyl-4*H*-1,2,4-triazin-5-one

A une suspension de 1,35 g d'hydrure de sodium (à 75% dans l'huile de vaseline) dans 25 $cm^3$ de diméthylformamide sous atmosphère d'argon et sous agitation, est ajoutée une solution de 10 g de 3-mercapto-6-(1-méthyl-1*H*-indol-3-yl)-4*H*-1,2,4-triazin-5-one, préparée à l'étape 1.1, dans 75 $cm^3$ de diméthylformamide. Après 1 h de réaction à une température voisine de 20˚C, le milieu réactionnel est additionné de 2,4 $cm^3$ d'iodure de méthyle puis il est agité 15 h à une température voisine de 20˚C. L'ajout de 50 $cm^3$ d'eau au mélange provoque l'apparition d'un précipité qui est collecté par filtration, rincé à trois reprises avec de l'eau et séché à l'air. Le solide obtenu est trituré dans de l'éther diisopropylique, collecté par filtration et séché sous pression réduite (2,7 kPa) pour donner 8,3 g de 6-(1-méthyl-1*H*-indol-3-yl)-3-méthylsulfanyl-4*H*-1,2,4-triazin-5-one sous la forme d'un solide jaune. Spectre RMN $^1H$ (300MHz ) - $\delta$ en ppm - dans le DMSO-d6 : 2,56 (s, 3H) ; 3,90 (s, 3H) ; de 7,17 à 7,35 (m, 2H) ; 7,56 (d large, J = 7,5 Hz, 1H) ; 8,33 (d large, J

= 7,5 Hz, 1H) ; 8,73 (s, 1H) ; de 13,6 à 14,1 (m très étalé, 1H)

1.3 6-(1-méthyl-1*H*-indol-3-yl)-3-(3-pipéridin-1-yl-propylamino)-4*H*-1,2,4-triazin-5-one

Un mélange sous atmosphère d'argon de 780 mg de 6-(1-méthyl-1*H*-indol-3-yl)-3-méthylsulfanyl-4*H*-1,2,4-triazin-5-one, préparée à l'étape 1.2, et de 1 g de 3-pipéridin-1-yl-propylamine est chauffé sous agitation 24 h à 130°C puis il est refroidi à une température voisine de 20°C et jeté dans de l'eau glacée. Le précipité formé se solubilise par l'addition d'acide chlorhydrique 1 N. Le milieu est ré-alcalinisé par ajout d'hydroxyde de sodium 1 N. Le solide qui précipite est collecté par filtration et cristallisé dans du méthanol. Après collecte par filtration et séchage des cristaux sous pression réduite (2,7 kPa), on obtient 260 mg de 6-(1-méthyl-1*H*-indol-3-yl)-3-(3-pipéridin-1-yl-propylamino)-4*H*-1,2,4-triazin-5-one sous la forme d'un solide gris. Spectre de masse (IC): m/z=367 ($MH^+$, pic de base). Spectre RMN $^1H$ (300MHz ) - $\delta$ en ppm - dans le DMSO-d6 :1,44 (m, 2H) ; 1,60 (m, 4H) ; 1,71 (m, 2H) ; de 2,29 à 2,42 (m, 6H) ; 3,26 (m, 2H) ; 3,86 (s, 3H) ; 7,10 (m étalé, 1H); 7,16 (t large, J = 7,5 Hz, 1H) ; 7,25 (t large, J = 7,5 Hz, 1H) ; 7,51 (d large, J = 7,5 Hz, 1H) ; 8,34 (d large, J = 7,5 Hz, 1H); 8,63 (s, 1H).

## Exemple 2

**3-(3-diéthylamino-propylamino)-6-(1-méthyl-1*H*-indol-3-yl)-4*H*-1,2,4-triazin-5-one** (composé n°1)

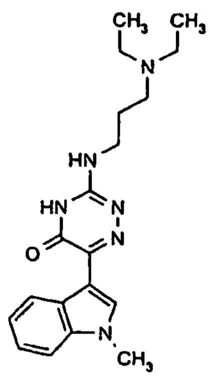

La 3-(3-diéthylamino-propylamino)-6-(1-méthyl-1*H*-indol-3-yl)-4*H*-1,2,4-tlazin-5-one est préparée suivant un protocole identique à celui décrit dans l'exemple 1, en remplaçant, à l'étape 1.3, la 3-pipéridin-1-yl-propylamine par la *N*1,*N*1-diéthyl-propane-1,3-diamine. Spectre RMN $^1H$ (400MHz ) - $\delta$ en ppm - dans le DMSO-d6 ) référencé à 2,50 ppm : 0,98 (t, J = 6,5 Hz, 6H); 1,66 (m, 2H) ; de 2,40 à 2,57 (m partiellement masqué, 6H) ; 3,27 (m partiellement masqué, 2H) ; 3,85 (s, 3H) ; 7,08 (t large, J = 6,5 Hz, 1H) ; 7,18 (t, J = 8,0 Hz, 1H) ; 7,24 (t, J = 8,0 Hz, 1H) ; 7,49 (d, J = 8,0 Hz, 1H); 8,32 (d, J = 8,0 Hz, 1H); 8,60 (s, 1H).

## Exemple 3

**3-(3-diéthylamino-propylamino)-6-phényl-4*H*-1,2,4-triazin-5-one** (composé n°20)

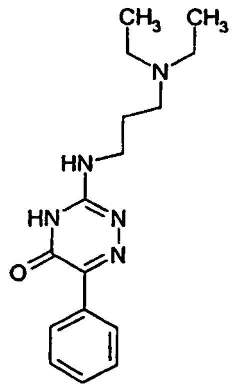

3.1 3-mercapto-6-phényl-4*H*-1,2,4-triazin-5-one

Une suspension de 10 g d'acide oxo-phényl-acétique et de 7 g de thiosemicarbazide dans 150 $cm^3$ d'eau est additionnée de 0,88 $cm^3$ d'acide chlorhydrique 12 N et le mélange est chauffé sous agitation 2,5 h au reflux du milieu réactionnel. Après refroidissement du mélange dans un bain d'eau glacée, le solide en suspension est collecté par filtration, rincé à l'eau et séché à l'air. Après avoir repris le solide jaune pâle obtenu (15,5 g) par 150 $cm^3$ d'eau et amené le milieu à un pH voisin de 14 à l'aide d'hydroxyde de sodium 1 N, on chauffe le mélange sous agitation 4 h au reflux du milieu réactionnel, on le refroidit dans un bain de glace, et on amène le pH à 4 par addition d'acide acétique. Le précipité résultant est collecté par filtration, rincé avec de l'eau et séché sur claie pendant 2 jours pour donner 11,3 g de 3-mercapto-6-phényl-4*H*-1,2,4-triazin-5-one sous la forme d'un solide jaune pâle. Spectre de masse (IE) : m/z=205 ($M^+$, pic de base), m/z=118 ($(M - C_2HNOS)^+$), m/z=104 ($C_7H_6N^+$).

3.2 3-méthylsulfanyl-6-phényl-4*H*-1,2,4-triazin-5-one

A une suspension de 1,96 g d'hydrure de sodium (à 75% dans l'huile de vaseline) dans 70 $cm^3$ de diméthylformamide sous atmosphère d'argon et sous agitation, est ajoutée une solution de 11,3 g de 3-mercapto-6-phényl-4*H*-1,2,4-triazin-5-one, préparée à l'étape 3.1, dans 120 $cm^3$ de diméthylformamide. Après 1 h de réaction à une température voisine de 20°C, le milieu réactionnel est additionné de 3,45 $cm^3$ d'iodure de méthyle, il est agité 15 h à une température voisine de 20°C puis jeté dans 200 $cm^3$ d'eau. Le refroidissement du mélange dans la glace provoque la cristallisation d'un composé qui est collecté par filtration et séché à l'air pour donner 7,6 g de 3-méthylsulfanyl-6-phényl-4*H*-1,2,4-triazin-5-one sous la forme d'un solide jaune pâle. Spectre de masse (IE): m/z=219 ($M^+$), m/z=172 ($(M - CH_3S)^+$), m/z=104 ($C_7H_6N^+$, pic de base).

3.3 3-(3-diéthylamino-propylamino)-6-phényl-4*H*-1,2,4-triazin-5-one

Un mélange sous atmosphère d'argon de 900 mg de 3-méthylsulfanyl-6-phényl-4*H*-1,2,4-triazin-5-one, préparée à l'étape 3.2, et de 1,65 $cm^3$ de *N*1,*N*1-diéthyl-propane-1,3-diamine est chauffé sous agitation 15 h à 100°C puis il est refroidi à une température voisine de 20°C et jeté dans de l'eau glacée. Le précipité formé par acidification du milieu à pH voisin de 4 avec de l'acide chlorhydrique 1 N est éliminé par filtration. Le filtrat est amené à pH voisin de 11 par addition de soude 1 N et il est extrait par deux fois avec du dichlorométhane. La phase aqueuse résultante est neutralisée à l'aide d'acide chlorhydrique 1 N et elle est extraite par deux fois avec du dichlorométhane. Les phases organiques sont rassemblées, séchées sur sulfate de magnésium, filtrées et concentrées sous pression réduite (2,7 kPa) pour donner une huile jaune (170 mg) qui est purifiée par chromatographie-flash sur alumine CTB1 sous pression d'argon (50 kPa) [éluant : dichlorométhane / méthanol (95 / 5 en volumes)]. Après concentration des fractions sous pression réduite, on obtient 115 mg de 3-(3-diéthylamino-propylamino)-6-phényl-4*H*-1,2,4-triazin-5-one sous la forme d'un solide crème. Spectre de masse (IC): m/z=302 ($MH^+$, pic de base). Spectre RMN $^1H$ (300MHz ) -, $\delta$ en ppm - dans le DMSO-d6 + 1 goutte d'acide acétique-d4 : 1,20 (t, J = 7,5 Hz, 6H) ; de 1,80 à 1,95 (m masqués, 2H) ; de 3,05 à 3,19 (m, 6H) ; 3,35 (t, J = 6,5 Hz, 2H) ; 7,41 (m, 3H) ; 7,96 (m, 2H).

**Exemple 4**

**3-(2-diéthylamino-éthylamino)-6-(1-méthyl-1*H*-indol-3-yl)-4*H*-1,2,4-triazin-5-one** (composé n°17)

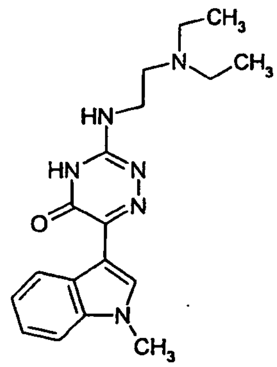

Un mélange sous atmosphère d'argon de 1 g de 6-(1-méthyl-1*H*-indol-3-yl)-3-méthylsulfanyl-4*H*-1,2,4-triazin-5-one, préparée selon l'étape 1.2 de l'exemple 1, et de 1,3 $cm^3$ de *N*1,*N*1-diéthyl-éthane-1,2-diamine est chauffé sous agitation 15 h à 130°C puis il est refroidi à une température voisine de 20°C. Le brut réactionnel est purifié par chromatographie-flash sur alumine CTB1 sous pression d'argon (50 kPa) [éluant : dichlorométhane puis dichlorométhane / méthanol (98 / 2 en volumes)]. Après concentration des fractions sous pression réduite (2,7 kPa), on obtient un résidu qui est trituré dans de l'éther diisopropylique, collecté par filtration et séché sous pression réduite pour donner 150 mg de 3-(2-diéthylamino-éthylamino)-6-(1-méthyl-1*H*-indol-3-yl)-4*H*-1,2,4-triazin-5-one sous la forme d'un solide crème. Spectre de masse (IE): m/z=340 ($M^+$), m/z=241 ((M - $C_6H_{13}N)^+$), m/z=86 ($C_5H_{12}N^+$, pic de base). Spectre RMN $^1H$ (300MHz ) - δ en ppm - dans le DMSO-d6 + 1 goutte d'acide acétique-d4 : 1,10 (m, 6H) ; de 2,72 à 2,90 (m, 6H) ; 3,42 (m, 2H) ; 3,85 (s, 3H) ; 7,18 (t large, J = 7,5 Hz, 1H); 7,26 (t large, J = 7,5 Hz, 1H) ; 7,52 (d large, J = 7,5 Hz, 1H) ; 8,32 (d large, J = 7,5 Hz, 1H) ; 8,62 (s, 1H).

**Exemple 5**

**3-(3-diméthylamino-propylamino)-6-(1-méthyl-1*H*-indol-3-yl)-4*H*-1,2,4-triazin-5-**one (composé n°14)

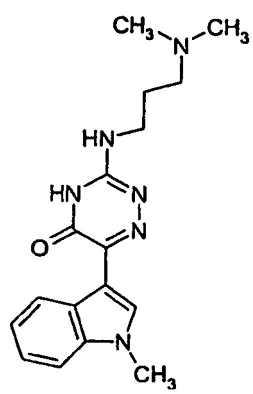

Un mélange sous atmosphère d'argon de 1 g de 6-(1-méthyl-1*H*-indol-3-yl)-3-méthylsulfanyl-4*H*-1,2,4-triazin-5-one, préparée selon l'étape 1.2 de l'exemple 1, et de 1,15 $cm^3$ de *N*1,*N*1-diméthyl-propane-1,3-diamine est chauffé sous agitation 15 h à 130°C puis il est refroidi à une température voisine de 20°C, additionné d'acide chlorhydrique 1 N de façon à ce que le pH du milieu soit voisin de 4 et extrait par du dichlorométhane qui est écarté. La phase aqueuse P est amenée à un pH voisin de 8 par addition de soude 1 N puis elle est extraite par deux fois avec du dichlorométhane. La phase organique est séchée sur sulfate de magnésium et concentrée sous pression réduite (2,7 kPa). L'huile résiduelle

obtenue est purifiée par chromatographie-flash sur alumine CTB1 sous pression d'argon (50 kPa) [éluant : dichlorométhane puis dichlorométhane / méthanol (98 / 2 en volumes)]. Après concentration des fractions sous pression réduite, on obtient un solide R1 (250 mg). La phase aqueuse P est alcalinisée jusqu'à pH voisin de 10 par ajout de soude 1 N et elle est extraite par deux fois avec du dichlorométhane. La phase organique est séchée sur sulfate de magnésium et concentrée sous pression réduite (2,7 kPa) pour donner un solide R2 (330 mg). Les solides R1 et R2 sont rassemblés, triturés à chaud dans de l'éther diisopropylique, collectés par filtration à chaud et séchés sous pression réduite pour donner 550 mg de 3-(3-diméthylamino-propylamino)-6-(1-méthyl-1*H*-indol-3-yl)-4*H*-1,2,4-triazin-5-one sous la forme d'un solide jaune pâle. Spectre de masse (IE): m/z=326 ($M^+$), m/z=282 (($M - C_2H_6N)^+$), m/z=156 ($C_{11}H_{10}N^+$), m/z=58 ($C_3H_8N^+$, pic de base). Spectre RMN $^1$H (300MHz ) - δ en ppm - dans le DMSO-d6 : 1,69 (m, 2H); 2,19 (s, 6H) ; 2,32 (t, J = 6,5 Hz, 2H) ; 3,29 (m, 2H) ; 3,85 (s, 3H) ; 7,08 (t très large, J = 6,0 Hz, 1H) ; 7,17 (t large J = 7,5 Hz, 1H) ; 7,26 (t large, J = 7,5 Hz, 1H); 7,51 (d large, J = 7,5 Hz, 1H) ; 8,33 (d large, J = 7,5 Hz, 1H) ; 8,62 (s, 1H).

**Exemple 6**

**6-(1-méthyl-1*H*-indol-3-yl)-3-(3-pyrrolidin-1-yl-propylamino)-4*H*-1,2,4-triazin-5-one** (composé n°8)

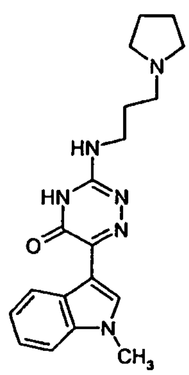

Un mélange sous atmosphère d'argon de 1,1 g de 6-(1-méthyl-1*H*-indol-3-yl)-3-méthylsulfanyl-4*H*-1,2,4-triazin-5-one, préparée selon l'étape 1.2 de l'exemple 1, et de 1,5 cm$^3$ de 3-pyrrolidin-1-yl-propylamine est chauffé sous agitation 15 h à 150°C puis il est refroidi à une température voisine de 20°C, trituré dans de l'acétate d'éthyle et filtré. Le résidu est repris par 10 cm$^3$ d'éthanol et le mélange est porté au reflux. La suspension est filtrée à chaud, le résidu est rincé par deux fois avec de l'éthanol et séché sous pression réduite (2,7 kPa) pour donner 690 mg de 6-(1-méthyl-1*H*-indol-3-yl)-3-(3-pyrrolidin-1-yl-propylamino)-4*H*-1,2,4-triazin-5-one sous la forme d'un solide beige. Spectre de masse (IE): m/z=352 ($M^+$), m/z=282 (($M - C_4H_8N^+$), m/z=156 ($C_{11}H_{10}N^+$), m/z=110 ($C_7H_{12}N^+$, pic de base), m/z=84 ($C_5H_{10}N^+$). Spectre RMN $^1$H (300MHz) - δ en ppm - dans le DMSO-d6 : De 1,64 à 1,82 (m, 6H) ; de 2,44 à 2,55 (partiellement masqués, 6H) ; 3,30 (masqués, 2H) ; 3,86 (s, 3H) ; 7,05 (t très large, J = 6,0 Hz, 1H) ; 7,16 (t large, J = 7,5 Hz, 1H) ; 7,25 (t large, J = 7,5 Hz, 1H) ; 7,50 (d large, J = 7,5 Hz, 1H) ; 8,32 (d large, J = 7,5 Hz, 1H) ; 8,63 (s, 1H).

**Exemple 7**

**6-(1-méthyl-1*H*-indol-3-yl)-3-[2-(1-méthyl-pyrrolidin-2-yl)-éthylamino]-4*H*-1,2,4-**triazin-5-one (composé n°15)

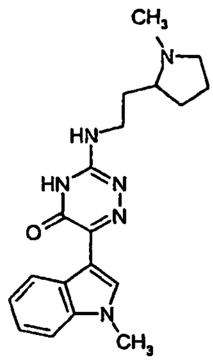

Un mélange sous atmosphère d'argon de 1,1 g de 6-(1-méthyl-1*H*-indol-3-yl)-3-méthylsulfanyl-4*H*-1,2,4-triazin-5-one, préparée selon l'étape 1.2 de l'exemple 1, et de 1,9 $cm^3$ de 2-(1-méthyl-pyrrolidin-2-yl)-éthylamine est chauffé sous agitation 15 h à 140°C puis il est refroidi à une température voisine de 20°C, repris par de l'acétate d'éthyle et de l'eau, et agité 15 min à une température voisine de 20°C. La phase organique est décantée, lavée par trois fois avec de l'eau et une fois avec de la saumure, puis elle est séchée sur sulfate de magnésium et concentrée sous pression réduite (2,7 kPa) pour donner une huile orangée (850 mg) qui est purifiée par chromatographie-flash sur alumine CTB1 sous pression d'argon (50 kPa) [éluant : acétate d'éthyle / méthanol (97 / 3 en volumes)]. Après évaporation des fractions sous pression réduite (2,7kPa), on obtient un solide qui est purifié une seconde fois par chromatographie-flash sur alumine CTB1 sous pression d'argon (50 kPa) [éludant : acétate d'éthyle / méthanol (90 / 10 en volumes)]. La concentration des fractions appropriées sous pression réduite (2,7 kPa) fournit un résidu qui est trituré dans de l'éther diisopropylique, collecté par filtration et séché sous pression réduite (2,7 kPa) pour donner 80 mg de 6-(1-méthyl-1*H*-indol-3-yl)-3-[2-(1-méthyl-pyrrolidin-2-yl)-éthylamino]-4*H*-1,2,4-triazin-5-one sous la forme d'un solide beige. Spectre de masse (IE): m/z=352 ($M^+$), m/z=156 ($C_{11}H_{10}N^+$), m/z=110 ($C_7H_{12}N^+$, pic de base), m/z=84 ($C_5H_{10}N^+$). Spectre RMN $^1H$ (300MHz ) - δ en ppm - dans le DMSO-d6 + 1 goutte d'acide acétique -d4 : de 1,65 à 2,40 (m, 6H) ; 2,84 (s, 3H) ; 3,10 (m, 1H) ; de 3,25 à 3,45 (m, 3H) ; 3,54 (m, 1H) ; 3,85 (s, 3H) ; 7,17 (t large, J = 7,5 Hz, 1H); 7,25 (t large, J = 7,5 Hz, 1H) ; 7,50 (d large, J = 7,5 Hz, 1H) ; 8,30 (d large, J = 7,5 Hz, 1H) ; 7,61 (s, 1H)

## Exemple 8

**6-(1*H*-indol-3-yl)-3-(3-pyrrolidin-1-yl-propylamino)-4*H*-1,2,4-triazin-5-one** (composé n°10)

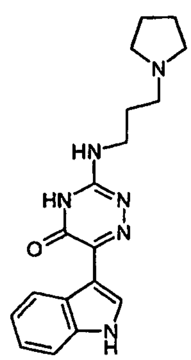

8.1 iodohydrate de S-méthyl-thiosemicarbazide

A une solution de 20 g de thiosemicarbazide dans 70 $cm^3$ de méthanol sous agitation sont ajoutés goutte à goutte, à une température voisine de 20°C, 14 $cm^3$ d'iodure de méthyle. L'agitation est poursuivie 3,5 h à la même température puis le milieu réactionnel est concentré à sec sous pression réduite (2,7 kPa). Le résidu est trois fois successivement trituré dans du dichlorométhane, collecté par filtration et séché sous pression réduite (2,7 kPa), pour donner 47,9 g d'iodohydrate de S-méthyl-thiosemicarbazide sous la forme d'un solide. Spectre de masse (IE): m/z=128

($IH^+$, pic de base), m/z=105 ($M^+$), m/z=58 ((M - $CH_3S)^+$).

8.2 iodhydrate de *N*-(3-pyrrolidin-1-yl-propyl)-hydrazinecarboximidamide

Un mélange de 1,99 g d'iodohydrate de S-méthyl-thiosemicarbazide, préparé à l'étape 8.1, et de 1 g de 3-pyrrolidin-1-yl-propylamine dans 30 $cm^3$ d'éthanol est agité 5 jours à une température voisine de 20˚C. Le milieu réactionnel est concentré à sec sous pression réduite (2,7 kPa), trituré dans de l'éther éthylique, filtré et séché sous pression réduite (2,7 kPa) pour donner 2,3 g d'iodohydrate de *N*-(3-pyrrolidin-1-yl-propyl)-hydrazinecarboximidamide sous la forme d'un solide. Spectre de masse (IC): m/z=186 ($MH^+$), m/z=129 ((M - $CH_4N_3$ + 2H$)^+$, pic de base).

8.3 6-(1*H*-indol-3-yl)-3-(3-pyrrolidin-1-yl-propylamino)-4*H*-1,2,4-triazin-5-one

Une solution de 0,55 g d'acide (1*H*-indol-3-yl)-oxo-acétique et de 1 g d'iodohydrate de N-(3-pyrrolidin-1-yl-propyl)-hydrazinecarboximidamide, préparé à l'étape 8.2, dans 3,5 $cm^3$ d'eau sont introduits dans un réacteur. Après avoir scellé le tube, on soumet le mélange pendant 10 min à une irradiation micro-onde discontinue telle que la température du milieu soit maintenue à 140˚C. Le mélange réactionnel est ensuite refroidi à une température voisine de 20˚C, additionné d'éthanol et concentré à sec sous pression réduite (2,7 kPa) pour donner un résidu qui est purifié par chromatographie-flash sur silice sous pression d'argon (50 kPa) [éluant : dichlorométhane / méthanol / ammoniaque 32% (90 / 10 / 1 puis 95 / 15 / 1,5 en volumes)]. Après concentration des fractions sous pression réduite (2,7 kPa), on obtient 108 mg de 6-(1*H*-indol-3-yl)-3-(3-pyrrolidin-1-yl-propylamino)-4*H*-1,2,4-triazin-5-one sous la forme d'un solide. Spectre de masse (ES) : m/z=339 ($MH^+$, pic de base). Spectre RMN $^1H$ (400MHz ) - $\delta$ en ppm - dans le DMSO-d6 : De 1,65 à 1,85 (m, 6H) ; 2,57 (m, 6H) ; 3,30 (m masqués, 2H) ; de 7,05 à 7,20 (m, 3H) ; 7,46 (d large, J = 8,5 Hz, 1H); 8,30 (d large, J = 8,0 Hz, 1H) ; 8,63 (d, J = 2,5 Hz, 1H) ; 11,45 (m large, 1H).

## Exemple 9

**6-(1*H*-indol-3-yl)-3-(3-pipéridin-1-yl-propylamino)-4*H*-1,2,4-triazin-5-one** (composé n˚9)

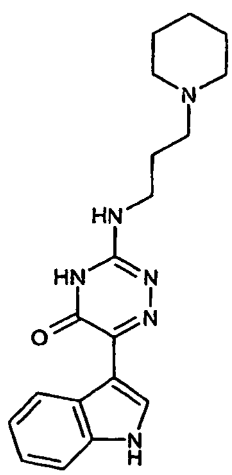

9.1 Iodohydrate de *N*-(3-pipéridin-1-yl-propyl)-hydrazinecarboximidamide

Un mélange de 2 g d'iodohydrate de *S*-méthyl-thiosemicarbazide, préparé selon l'étape 8.1 de l'exemple 8, et de 1,22 g de 3-pipéridin-1-yl-propylamine dans 30 $cm^3$ d'éthanol est agité 6 jours à une température voisine de 20˚C. Le milieu réactionnel est concentré à sec sous pression réduite (2,7 kPa), et à trois reprises successivement trituré dans de l'éther éthylique, filtré et séché sous pression réduite (2,7 kPa), pour donner 2,6 g d'iodohydrate de *N*-(3-pipéridin-1-yl-propyl)-hydrazinecarboximidamide sous la forme d'un solide. Spectre de masse (IE): m/z=199 ($M^+$), m/z=168 ((M - $N_2H_3)^+$), m/z=128 ($IH^+$, pic de base), m/z=98 ($C_6H_{12}N^+$).

9:2 6-(1*H*-indol-3-yl)-3-(3-pipéridin-1-yl-propylamino)-4*H*-1,2,4-triazin-5-one

Une solution de 0,55 g d'acide (1*H*-indol-3-yl)-oxo-acétique et de 1,04 g d'iodohydrate de N-(3-pipéridin-1-yl-propyl)-hydrazinecarboximidamide, préparé à l'étape 9.1, dans 3,5 $cm^3$ d'eau sont introduits dans un réacteur. Après avoir scellé le tube, on soumet le mélange pendant 10 min à une irradiation micro-onde discontinue telle que la température du milieu soit maintenue à 140°C. Le mélange réactionnel est ensuite refroidi à une température voisine de 20°C, additionné d'éthanol et évaporé à sec sous pression réduite (2,7 kPa) pour donner un résidu qui est purifié par chromatographie-flash sur silice sous pression d'argon (50 kPa) [éluant : dichlorométhane / méthanol / ammoniaque 32% (90 / 10 / 1 en volumes)]. Après concentration des fractions sous pression réduite (2,7 kPa), on obtient 35 mg de 6-(1*H*-indol-3-yl)-3-(3-pipéridin-1-yl-propylamino)-4*H*-1,2,4-triazin-5-one sous la forme d'un solide. Spectre de masse (ES) : m/z=353 ($MH^+$, pic de base). Spectre RMN $^1H$ (300MHz ) - $\delta$ en ppm - dans le DMSO-d6 : De 1,15 à 1,85 (m étalé, 6H) ; 1,95 (m, 2H) ; de 2,80 à 3,55 (m étalé, 8H) ; de 7,05 à 7,22 (m, 3H) ; 7,47 (d large, J = 8,0 Hz, 1H) ; 8,31 (d large, J = 8,0 Hz, 1H) ; 8,62 (d, J = 2,5 Hz, 1H) ; de 8,95 à 9,15 (m étalé, 1H) ; 11,45 (m large, 1H) ; 12,3 (m étalé, 1H).

## Exemple 10

**Chlorhydrate hydrate de 3-[(*S*)-(1-aza-bicyclo[2.2.2]oct-3-yl)amino]-6-(1*H*-indol-3-yl)-4*H*-1,2,4-triazin-5-one** (composé n°6)

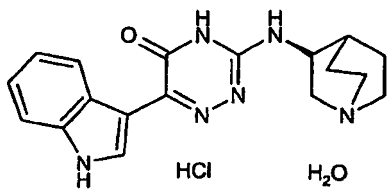

10.1 Iodohydrate de (*S*)-*N*-(1-aza-bicyclo[2.2.2]oct-3-yl)-hydrazinecarboximidamide

Un mélange de 2.77 g d'iodohydrate de *S*-méthyl-thiosemicarbazide, préparé selon l'étape 8.1 de l'exemple 8, et de 1,5 g de (*S*)-(1-aza-bicyclo[2.2.2]oct-3-yl)amine dans 25 $cm^3$ d'éthanol est agité 6 jours à une température voisine de 20°C. Le milieu réactionnel est concentré à sec sous pression réduite (2,7 kPa), et le résidu est trituré dans de l'éther éthylique, collecté par filtration et séché sous pression réduite (2,7 kPa), pour donner 3,2 g d'iodohydrate de (*S*)-*N*-(1-aza-bicyclo[2.2.2]oct-3-yl)-hydrazinecarboximidamide sous la forme d'un solide qui est utilisé sans autre purification à l'étape suivante.

10.2 Chlorhydrate hydrate de 3-[(*S*)-(1-aza-bicyclo[2.2.2]oct-3-yl)amino]-6-(1*H*-indol-3-yl)-4*H*-1,2,4-triazin-5-one

Une solution de 0,55 g d'acide (1*H*-indol-3-yl)-oxo-acétique et de 1,2 g d'iodohydrate de (*S*)-*N*-(1-aza-bicyclo [2.2.2]oct-3-yl)-hydrazinecarboximidamide, préparé à l'étape 10.1, dans 3 $cm^3$ d'eau sont introduits dans un réacteur. Après avoir scellé le tube, on soumet le mélange pendant 20 min à une irradiation micro-onde discontinue telle que la température du milieu soit maintenue à 140°C. Le mélange réactionnel est ensuite refroidi à une température voisine de 20°C, additionné de méthanol et évaporé à sec sous pression réduite (2,7 kPa) pour donner un résidu qui est purifié par chromatographie-flash sur silice sous pression d'argon (50 kPa) [éluant : dichlorométhane / méthanol / ammoniaque 32% (75 / 22 / 3 en volumes)]. Après concentration des fractions sous pression réduite (2,7 kPa), on obtient un solide qui est lavé successivement par du dichlorométhane et de l'éther éthylique, puis séché sous pression réduite (2,7 kPa) pour donner 74 mg de chlorhydrate hydrate de 3-[(*S*)-(1-aza-bicyclo[2.2.2]oct-3-yl)amino]-6-(1*H*-indol-3-yl)-4*H*-1,2,4-triazin-5-one sous la forme d'un solide jaune. Spectre de masse (IE) : m/z=336 ($M^+$, pic de base), m/z=252 ($(M - C_5H_{10}N)^{+\cdot}$), m/z=227 ($(M - C_7H_{11}N)^+$), m/z=109 ($C_7H_{11}N^{+\cdot}$), m/z=36 ($HCl^{+\cdot}$). Spectre RMN $^1H$ (400MHz ) - $\delta$ en ppm - dans le DMSO-d6 référencé à 2,50 ppm : 1,66 (m, 1H) ; 1,83 (m, 2H) ; 2,00 (m, 1H) ; 2,12 (m, 1H) ; 2,93 (m, 1H) ; de 3,00 à 3,19 (m, 4H) ; 3,54 (m, 1H) ; 4,10 (m, 1H) ; 7,10 (t, J = 8,0 Hz, 1H) ; 7,17 (t, J = 8,0 Hz, 1H) ; 7,46 (d, J = 8,0 Hz, 1H) ; 7,66 (d large, J = 7,0 Hz, 1H) ; 8,29 (d, J = 8,0 Hz, 1H) ; 8,60 (d, J = 2,0 Hz, 1H) ; 11,5 (s large, 1H) ; de 11,9 à 12,4 (m très étalé, 1H). $[\alpha]^{20}_D$ = -24.5 +/- 0.7° (c 0,42, méthanol).

## Exemple 11

**Chlorhydrate de 3-[(*R*)-(1-aza-bicyclo[2.2.2]oct-3-yl)amino]-6-(1*H*-indol-3-yl)-4*H*-1,2,4-triazin-5-one** (composé n°13)

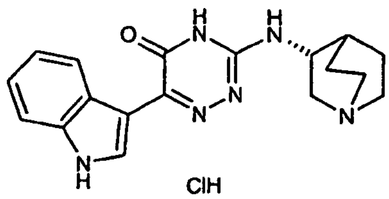

11.1 Iodohydrate de (*R*)-*N*-(1-aza-bicyclo[2.2.2]oct-3-yl)-hydrazinecarboximidamide

Un mélange de 1 g d'iodohydrate de *S*-méthyl-thiosemicarbazide, préparé selon l'étape 8.1 de l'exemple 8, de 0,89 g de dichlorhydrate de (*R*)-(1-aza-bicyclo[2.2.2]oct-3-yl)amine et de 1,2 $cm^3$ de triéthylamine dans 20 $cm^3$ d'éthanol est agité 6 jours à une température voisine de 20°C. Le milieu réactionnel est concentré à sec sous pression réduite (2,7 kPa), et le résidu est trituré dans de l'éther éthylique, collecté par filtration et séché sous pression réduite (2,7 kPa), pour donner 1,25 g d'iodohydrate de (R)-N-(1-aza-bicyclo[2.2.2]oct-3-yl)-hydrazinecarboximidamide sous la forme d'un solide. Spectre de masse (ES) : m/z=184 ($MH^+$), m/z=127 (($M - CH_4N_3 + 2H)^+$), m/z=102 (($M - C_5H_9N + 2H)^+$), pic de base).

11.2 Chlorhydrate de 3-[(*R*)-(1-aza-bicyclo[2.2.2]oct-3-yl)amino]-6-(1*H*-indol-3-yl)-4*H*-1,2,4-triazin-5-one

Une solution de 0,55 g d'acide (1*H*-indol-3-yl)-oxo-acétique et de 1,22 g d'iodohydrate de (*R*)-*N*-(1-aza-bicyclo[2.2.2]oct-3-yl)-hydrazinecarboximidamide, préparé à l'étape 11.1, dans 3 $cm^3$ d'eau sont introduits dans un réacteur. Après avoir scellé le tube, on soumet le mélange pendant 20 min à une irradiation micro-onde discontinue telle que la température du milieu soit maintenue à 140°C. Le mélange réactionnel est ensuite refroidi à une température voisine de 20°C, additionné de méthanol et évaporé à sec sous pression réduite (2,7 kPa) pour donner un résidu qui est purifié par chromatographie-flash sur silice sous pression d'argon (50 kPa) [éluant : dichlorométhane / méthanol / ammoniaque 32% (75 / 22/ 3 en volumes)]. Après concentration des fractions sous pression réduite (2,7 kPa), on obtient un résidu qui est lavé successivement par du dichlorométhane et de l'éther éthylique, puis séché sous pression réduite (2,7 kPa). Le solide obtenu est traité sur une cartouche BOND ELUT VARIAN contenant 2 g de phase SCX conditionnée préalablement avec du méthanol (éluant : méthanol puis méthanol ammoniacal 2 N) et les fractions appropriées sont évaporées sous pression réduite (2,7 kPa) pour donner un résidu qui est dissous dans de l'éthanol et additionné d'une solution 1 N d'acide chlorhydrique dans l'éther éthylique. Le précipité formé est collecté par filtration, lavé par de l'éther éthylique et séché sous pression réduite (2,7 kPa). On obtient 60 mg de chlorhydrate de 3-[(*R*)-(1-aza-bicyclo[2.2.2]oct-3-yl)amino]-6-(1*H*-indol-3-yl)-4*H*-1,2,4-triazin-5-one sous la forme d'un solide orangé. Spectre de masse (ES) : m/z=337 ($MH^+$). Spectre RMN $^1H$ (400MHz ) - δ en ppm - dans le DMSO-d6 référencé à 2,50 ppm : 1,79 (m, 1H); 1,92 (m, 2H); 2,10 (m, 1H); 2,22 (m, 1H) ; 3,11 (m, 1H); de 3,17 à 3,32 (m, 4H) ; de 3,60 à 3,90 (m partiellement masqué, 1H) ; 4,22 (m, 1H) ; 7,11 (t, J = 8,0 Hz, 1H) ; 7,18 (t, J = 8,0 Hz, 1H) ; 7,46 (d, J = 8,0 Hz, 1H) ; 7,90 (d large, J = 6,0 Hz, 1H) ; 8,30 (d, J = 8,0 Hz, 1H) ; 8,60 (d, J = 2,0 Hz, 1H) ; 9,92 (s large, 1H) ; 11,5 (s large, 1H) ; de 12,2 à 12,5 (m étalé, 1H).

## Exemple 12

**6-(furan-3-yl)-3-(3-diéthylaminopropylamino)-4*H*-1,2,4-triazin-5-one** (composé n°23)

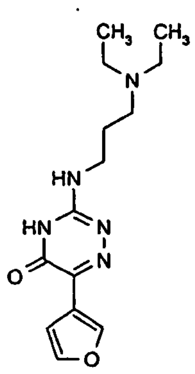

12.1 iodohydrate de *N*-amino-*N'*-(3-diéthylaminopropyl)-guanidine

Un mélange de 18,38 g d'iodhydrate de *S*-méthyl-thiosemicarbazide, préparé selon l'étape 8.1 de l'exemple 8, et de 9,45 $cm^3$ de 3-(diéthylamino)propylamine dans 180 $cm^3$ d'éthanol est agité à une température voisine de 20°C pendant 48 h. La solution orange est concentrée sous pression réduite (2,7 kPa). Le résidu est repris à l'éther éthylique et le surnageant est éliminé. On recommence deux fois cette opération puis on empâte le solide résiduel à l'éther éthylique et on l'essore pour donner 17,36 g d'iodohydrate de *N*-amino-*N'*-(3-diéthylaminopropyl)-guanidine.

12.2 6-(furan-3-yl)-3-(3-diéthylaminopropylamino)-4*H*-1,2,4-triazin-5-one

Un mélange de 473 mg d'iodohydrate de *N*-amino-*N'*-(3-diéthylamino-propyl)-guanidine, préparé à l'étape 12.1, et de 210 mg d'acide furan-3-yl-oxo-acétique dans 3 $cm^3$ d'eau, est chauffé pendant 10 min à 140°C au four à micro-ondes puis il est refroidi à une température voisine de 20°C et additionné de 15 $cm^3$ de dichlorométhane et de 5 $cm^3$ de soude 1 N. Après décantation, la phase organique est soutirée et la phase aqueuse est ré-extraite par deux fois avec 15 $cm^3$ de dichlorométhane. Les phases organiques sont réunies, lavées avec de l'eau, séchées sur sulfate de magnésium, filtrées et concentrées à sec sous pression réduite (2,7 kPa). On obtient 1,4 g d'un produit brut qui est purifié par chromatographie sur silice 60 (40-63 $\mu$m) sous pression d'argon (50 kPa) [éluant : dichlorométhane puis dichlorométhane / méthanol / ammoniaque 32% (90 / 9 / 1 en volumes)] pour donner 0,185 g d'un solide jaune pâle, qui est ensuite repris à l'éther, essoré, lavé avec un minimum d'éther, puis séché sous pression réduite (2,7 kPa) à 40°C. On obtient ainsi 0,155 g d'une poudre jaune pâle. Spectre de masse (ES) : m/z = 292 ($MH^+$) ; Spectre RMN 1H (300MHz ) - $\delta$ en ppm - dans le DMSO-d6 : 1,00 (t, J = 7,5 Hz, 6H) ; 1,67 (m, 2H) ; de 2,42 à, 2,60 (m, 6H) ; 3,27 (m, 2H) ; 6,84 (d, J = 2,0 Hz, 1H) ; 7,24 (m étalé, 1H) ; 7,74 (t, J = 2,0 Hz, 1H) ; 8,53 (d, J = 2,0 Hz, 1H).

**Exemple 13**

**6-(naphtalèn-1-yl)-3-(3-diéthylaminopropylamino*)-4H-*1,2,4-triazin-5-one** (composé n°21)

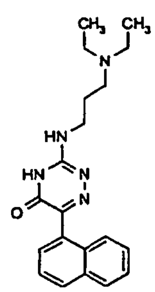

13.1 naphthalèn-1-yl-oxo-acétate de sodium

Un mélange biphasique de 0,912 g de naphthoylformate d'éthyle et de 4 $cm^3$ de soude 1 N dans 4 $cm^3$ d'eau

est chauffé à une température voisine de 70°C durant 2 h pendant lesquelles le milieu devient homogène puis il est filtré sur Acrodisc® Gelman 0,45 μm. Le filtrat est lyophilisé pour donner 0,855 g de naphthalèn-1-yl-oxo-acétate de sodium sous forme d'une poudre blanche.

13.2 6-(naphthalèn-1-yl)-3-(3-diéthylaminopropylamino)-4*H*-1,2,4-triazin-5-one

Un mélange de 640 mg d'iodohydrate de *N*-amino-*N*'-(3-diéthylaminopropyl)-guanidine, préparé à l'étape 12.1 de l'exemple 12, et de 0,44 g de naphthalèn-1-yl-oxo-acétate de sodium, préparé à l'étape 13.1, dans 0,5 $cm^3$ d'acide acétique et 2 $cm^3$ d'eau, est chauffé pendant 10 min à 140°C au four à micro-ondes puis il est refroidi à une température voisine de 20°C, dilué avec 15 $cm^3$ de dichlorométhane et 10 $cm^3$ de soude 1 N et décanté. La phase organique est soutirée et la phase aqueuse est ré-extraite par deux fois avec 15 $cm^3$ de dichlorométhane. Les phases organiques sont réunies, lavées avec de l'eau, séchées sur sulfate de magnésium, filtrées et concentrées sous pression réduite. On obtient 0,384 g d'un produit brut, qui est purifié par chromatographie sur silice 60 (40-63 μm) [éluant : dichlorométhane puis dichlorométhane / méthanol / ammoniaque ( 85/ 14/ 1 en volumes)] pour donner 0.058 g solide jaune pâle, qui est ensuite repris à l'éther, essoré, lavé avec un minimum d'éther et séché sous pression réduite (2,7 kPa) à 40°C. On obtient ainsi 0,048 g de 6-(naphthalèn-1-yl)-3-(3-diéthylaminopropylamino)-4*H*-1,2,4-triazin-5-one sous forme d'une poudre blanche. Spectre de masse (ES) : m/z = 352 ($MH^+$); Spectre RMN 1H (300MHz ) - δ en ppm - dans le DMSO-d6 : 1,17 (t, J = 7,5 Hz, 6H) ; 1,90 (m, 2H) ; de 2,40 à 3,00 (m partiellement masqués, 6H) ; 3,42 (m, 2H) ; 7,12 (m étalé, 1H) ; de 7,44 à 7,60 (m, 4H) ; 7,80 (d large, J = 7,5 Hz, 1H) ; 7,98 (m, 2H).

**Exemple 14**

**Iodohydrate de 6-(1-méthyl-5-méthoxy-indol-3-yl)-3-(3-diéthylaminopropylamino)-4*H*-1,2,4-triazin-5-one** (composé n°22)

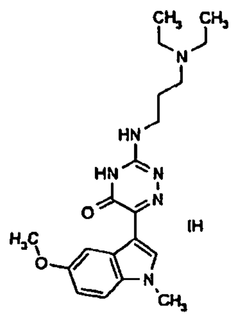

Un mélange de 472 mg d'iodohydrate de *N*-amino-*N*'-(3-diéthylamino-propyl)-guanidine, préparé à l'étape 12.1 de l'exemple 12, et de 349 mg d'acide (5-méthoxy-1-méthyl-1*H*-indol-3-yl)-oxo-acétique dans 3 $cm^3$ d'eau et 0,5 $cm^3$ de dioxane est chauffé pendant 10 min à 140°C au four à micro-ondes puis il est refroidi à une température voisine de 20°C et évaporé à sec sous pression réduite (2,7 kPa). On obtient 810 mg d'un solide marron qui est purifié par chromatographie sur silice 60 (40-63 μm) [éluant: dichlorométhane puis dichlorométhane / méthanol / ammoniaque 32% ( 85/ 14 / 1 en volumes)] pour donner 0,157 g d'un solide jaune qui est ensuite repris dans du méthanol, essoré, lavé avec un minimum de méthanol et séché sous pression réduite (2,7 kPa) à 40°C. On obtient ainsi 0,087 g d'iodohydrate de 6-(1-méthyl-5-méthoxy-indol-3-yl)-3-(3-diéthylaminopropylamino)-4*H*-1,2,4-triazin-5-one sous forme d'une poudre jaune. Spectre de masse (ES) : m/z = 385 ($MH^+$) ; Spectre RMN 1H (300MHz ) - δ en ppm - dans le DMSO-d6 : 1,22 (t, J = 7,5 Hz, 6H) ; 1,92 (m, 2H) ; 3,13 (m, 6H) ; 3,34 (m masqué, 2H) ; 3,81 (s, 3H) ; 3,83 (s, 3H) ; 6,92 (dd, J = 2,5 et 9,0 Hz, 1H) ; 7,13 (m étalé, 1H) ; 7,43 (d, J = 9,0 Hz, 1H) ; 7,84 (d, J = 2,5 Hz, 1H) ; 8,60 (s, 1H) ; 9,15 (m étalé, 1H) ; 12,3 (m très étalé, 1H).

**Exemple 15**

**6-(1-phényl-indol-3-yl)-3-(3-diéthylaminopropylamino)-4*H*-1,2,4-triazin-5-one** (composé n°11)

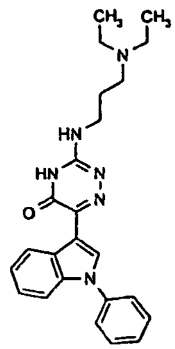

15.1 1-phényl-1*H*-indole

Un mélange de 1,17 g d'indole, 1,6 $cm^3$ de bromobenzène, 112 mg d'acétate de palladium, 333 mg de 1,1'-bis(diphénylphosphino)ferrocène et 1,44 g de tertiobutylate de sodium dans 20 $cm^3$ de toluène est chauffé pendant 48 h au reflux. La suspension de couleur marron foncé est diluée avec 50 $cm^3$ d'acétate d'éthyle et 10 $cm^3$ d'eau. Les insolubles sont filtrés et les deux phases sont décantées. La phase aqueuse est ré-extraite à l'acétate d'éthyle, les phases organiques réunies sont lavées avec une solution saturée de chlorure d'ammonium, séchées sur sulfate de sodium, filtrées et concentrées à sec sous pression réduite (2,7 kPa). L'huile marron (1,85 g) obtenue est purifiée par une chromatographie sur silice 60 (40-63 μm) [éluant : heptane / acétate d'éthyle (90 / 10 en volumes)] pour donner 0,375 g de 1-phényl-1*H*-indole sous la forme d'un solide jaune pâle.

15.2 acide oxo-(1-phényl-1*H*-indol-3-yl)-acétique

Une solution de 0,368 $cm^3$ de chlorure d'oxalyle dans 2 $cm^3$ d'éther est introduite goutte à goutte, à une température voisine de 0°C, à une solution dans 16 $cm^3$ d'éther de 0,35 g de 1-phényl-1*H*-indole, préparé à l'étape 15.1. On laisse revenir le milieu réactionnel à une température voisine de 20°C et on agite le mélange pendant 3 h à cette température. Le milieu réactionnel est ensuite amené à pH 8 avec de la soude 1 N et il est extrait par de l'acétate d'éthyle. La phase aqueuse est acidifiée jusqu'à pH 2 avec de l'acide chlorhydrique concentré. Les précipités formés sont collectés par filtration, lavés avec de l'eau et de l'éther, et séchés sous pression réduite (2,7 kPa) à 40°C pour donner 0,352 g d'acide oxo-(1-phényl-1*H*-indol-3-yl)-acétique.

15.3 6-(1-phényl-indol-3-yl)-3-(3-diéthylaminopropylamino)-4*H*-1,2,4-triazin-5-one

Un mélange de 458 mg d'iodohydrate de *N*-amino-*N*'-(3-diéthylamino-propyl)-guanidine, préparé à l'étape 12.1 de l'exemple 12, et de 340 mg d'acide oxo-(1-phényl-1*H*-indol-3-yl)-acétique, préparé à l'étape 15.2, dans 4 $cm^3$ d'eau est chauffé pendant 10 min à 140°C au four à micro-ondes puis il est refroidi à une température voisine de 20°C, dilué avec 15 $cm^3$ de dichlorométhane et 5 $cm^3$ de soude 1 N, et il est décanté. La phase organique est soutirée et la phase aqueuse est ré-extraite par deux fois avec 15 $cm^3$ de dichlorométhane. Les phases organiques réunies sont lavées avec de l'eau, séchées sur sulfate de magnésium, filtrées et concentrées à sec sous pression réduite (2,7 kPa). On obtient 0,3 g d'un résidu jaune qui est purifié par une chromatographie sur silice 60 (40-63 μm) [éluant dichlorométhane / méthanol / ammoniaque 32% ( 90 / 9 / 1 en volumes)] pour donner 0,135 g de 6-(1-phényl-indol-3-yl)-3-(3-diéthylaminopropylamino)-4*H*-1,2,4-triazin-5-one sous forme d'un solide jaune pâle. Spectre de masse (ES) : m/z = 417 ($MH^+$), 344 ($M-C_4H_{11}N+H^+$). Spectre RMN 1H (300MHz ) - δ en ppm - dans le DMSO-d6 : 1,01 (t, J = 7,5 Hz, 6H) ; 1,70 (m, 2H) ; de 2,44 à 2,55 (m partiellement masqué, 6H) ; 3,31 (m, 2H) ; de 7,14 à 7,34 (m, 3H) ; de 7,45 à 7,70 (m, 6H) ; 8,45 (dd, J = 1,5 et 7,5 Hz, 1H) ; 8,85 (s, 1H).

## <u>Exemple 16</u>

**6-(indol-3-yl)-3-(3-diéthylaminopropylamino)-4*H*-1,2,4-triazin-5-one** (composé n°7)

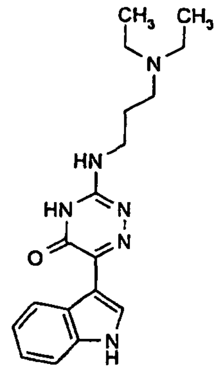

16.1 acide (1*H*-indol-3-yl)-oxo-acétique

Un mélange de 610 mg d'indolyl-3-glyoxylate de méthyle et de 3,3 $cm^3$ de soude 1 N dans 3,3 $cm^3$ d'eau est chauffé pendant 1 h à 80°C, puis il est refroidi dans un bain de glace et additionné de 3,5 $cm^3$ d'acide chlorhydrique 1 N. Le mélange est extrait par de l'acétate d'éthyle. Les phases organiques réunies sont séchées sur sulfate de sodium, filtrées et concentrées à sec sous pression réduite (2,7 kPa) pour donner 554 mg d'acide (1*H*-indol-3-yl)-oxo-acétique sous la forme d'un solide jaune.

16.2 6-(indol-3-yl)-3-(3-diéthylaminopropylamino)-4*H*-1,2,4-triazin-5-one

Un mélange de 350 mg d'iodohydrate de *N*-amino-*N'*-(3-diéthylamino-propyl)-guanidine, préparé à l'étape 12.1 de l'exemple 12, et de 189 mg d'acide (1*H*-indol-3-yl)-oxo-acétique, préparé à l'étape 16.1, dans 4 $cm^3$ d'eau est chauffé pendant 15 min à 140°C au four à micro-ondes puis il est refroidi à une température voisine de 20°C et dilué par de l'acétate d'éthyle et de l'eau. La phase aqueuse est extraite par de l'acétate d'éthyle, additionnée d'acétate d'éthyle puis alcalinisée avec de la soude 2 N. La phase organique est soutirée et la phase aqueuse est ré-extraite par de l'acétate d'éthyle. Les phases organiques réunies sont séchées sur sulfate de magnésium, filtrées et concentrées à sec sous pression réduite (2,7 kPa). On triture le résidu dans l'acétate d'éthyle, on le collecte par filtration et on essore le solide (83 mg) qui est empâté dans le méthanol puis essoré. Le produit obtenu (50 mg) est purifié par chromatographie sur silice, [éluant dichlorométhane / méthanol / ammoniaque 32% ( 90 / 9 / 1, 90 / 10 / 0,5 puis 85 / 15 / 1 en volumes)] pour donner 0,044 g de 6-(indol-3-yl)-3-(3-diéthylaminopropylamino)-4*H*-1,2,4-triazin-5-one sous la forme d'un solide jaune pâle. Spectre de masse (ES) : m/z = 341 ($MH^+$) ; Spectre RMN 1H (400MHz ) - δ en ppm - dans le DMSO-d6 : 1,01 (t, J = 7,5 Hz, 6H) ; 1,69 (m, 2H) ; de 2,38 à 2,55 (m partiellement masqués, 6H) ; 3,30 (partiellement masqués, 2H) ; de 7,03 à 7,21 (m, 3H) ; 7,46 (d large, J = 8,0 Hz, 1H) ; 8,32 (d large, J = 8,0 Hz, 1H) ; 8,64 (d, J = 2,0 Hz, 1H) 11,5 (m large, 1H).

## Exemple 17

**6-(benzothiophèn-3-yl)-3-(3-diéthylaminopropylamino)-4*H*-1,2,4-triazin-5-one** (composé n°12)

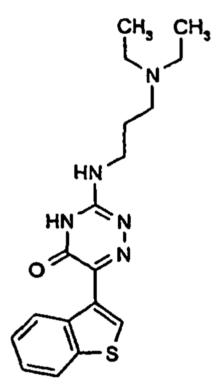

17.1 benzo[b]thiophèn-3-yl-oxo-acétate d'éthyle

A une solution, sous azote et refroidie à -10°C, de 8,34 $cm^3$ de chlorure d'oxalyle dans 140 $cm^3$ de dichlorométhane sont ajoutés successivement 10,54 g de chlorure d'aluminium, puis ,en 2h, une solution de 10 g de benzothiophène dans 100 $cm^3$ de dichlorométhane. La température est maintenue à -10°C pendant toute la durée de l'addition puis on poursuit l'agitation pendant environ 1 h à une température voisine de 20°C. Le milieu réactionnel est ensuite versé sur de l'eau glacée et additionné d'une solution saturée de tartrate double de potassium et de sodium qui provoque la précipitation des sels d'aluminium. La suspension est filtrée sur Hyflosupercel® Kieselghur et décantée. La phase organique est soutirée et la phase aqueuse est ré-extraite par du dichlorométhane. Les phases organiques réunies sont lavées à l'eau, séchées sur sulfate de sodium et concentrées à sec sous pression réduite (2,7 kPa). Le résidu est purifié par une chromatographie sur la silice, [éluant : dichlorométhane / heptane ( 70 / 30 en volumes)] pour donner 10,78 g de benzo[b]thiophèn-3-yl-oxo-acétate d'éthyle sous forme d'une huile orange.

17.2 6-(benzothiophèn-3-yl)-3-(3-diéthylaminopropylamino)-4*H*-1,2,4-triazin-5-one

Un mélange de 469 mg de benzo[b]thiophèn-3-yl-oxo-acétate d'éthyle, préparé à l'étape 17.1, et de 2 $cm^3$ de soude 1 N dans 2 $cm^3$ d'eau est agité 15 min à une température voisine de 20°C puis chauffé à 70°C pendant 30 min. Le milieu réactionnel est ramené à une température voisine de 20°C puis il transféré dans un récipient pour micro-ondes. On ajoute au milieu 700 mg d'iodohydrate de N-amino-N'-(3-diéthylamino-propyl)-guanidine, préparé à l'étape 12.1 de l'exemple 12, puis on chauffe à 140°C pendant 10 min. Le milieu réactionnel est concentré sous pression réduite (2,7 kPa) puis il est purifié par une chromatographie sur silice, [éluant : dichlorométhane / méthanol / ammoniaque 32% ( 90 / 9 / 1, 90 / 10 / 0,5 puis 85 / 15 / 1 en volumes)] pour donner 0,045 g de 6-(benzothiophèn-3-yl)-3-(3-diéthylaminopropylamino)-4*H*-1,2,4-triazin-5-one sous la forme d'un solide jaune pâle. Spectre de masse (ES) : m/z = 357 ($MH^+$) ; Spectre RMN 1H (400MHz ) - δ en ppm - dans le DMSO-d6 : 1,01 (t, J = 7,5 Hz, 6H); 1,70 (m, 2H) ; de 2,42 à 2,59 (m partiellement masqué, 6H) ; 3,30 (m, 2H) ; de 7,30 à 7,50 (m, 3H) ; 8,06 (d large, J = 8,0 Hz, 1H) ; 8,54 (d large, J = 8,0 Hz, 1H) ; 8,92 (s, 1H).

**Exemple 18**

**6-(4-méthoxyphényl)-3-(3-diéthylaminopropylamino)-4*H*-1,2,4-triazin-5-one** (composé n°18)

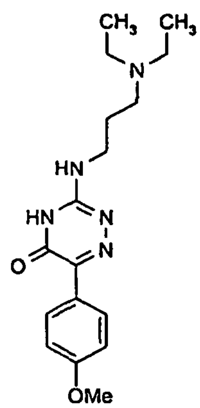

18.1 (4-méthoxy-phényl)-oxo-acétate de sodium

Un mélange de 1,041 g 4-méthoxybenzoate d'éthyle et de 5 $cm^3$ de soude 1 N dans 5 $cm^3$ d'eau est chauffé à 80°C pendant 1 h, puis il est refroidi et lyophilisé pour donner 1 g de (4-méthoxy-phényl)-oxo-acétate de sodium.

18.2 6-(4-méthoxyphényl)-3-(3-diéthylaminopropylamino)-4*H*-1,2,4-triazin-5-one

Un mélange de 700 mg d'iodohydrate de *N*-amino-*N*'-(3-diéthylamino-propyl)-guanidine, préparé à l'étape 12.1 de l'exemple 12, et de 400 mg de (4-méthoxy-phényl)-oxo-acétate de sodium préparé à l'étape 18.1, dans 4 $cm^3$ d'eau est chauffé pendant 15 min à 140°C au four à micro-ondes puis il est refroidi à une température voisine de 20°C. Le

milieu réactionnel est amené à sec pression réduite (2,7 kPa) et le résidu est purifié par une chromatographie sur silice [éluant : dichlorométhane / méthanol / ammoniaque 32% ( 90 / 9 / 1 en volumes)] pour donner un solide qui est empâté dans de l'acétate d 'éthyle et essoré. On obtient ainsi 0,092 g de 6-(4-méthoxyphényl)-3-(3-diéthylaminopropylamino)-4*H*-1,2,4-triazin-5-one sous la forme d'un solide blanc. Spectre de masse (ES) : m/z = 332 ($MH^+$), 259 ($M-C_4H_{11}N+H^+$) ; Spectre RMN 1H (300MHz ) - δ en ppm - dans le DMSO-d6 : 0,98 (t, J = 7,5 Hz, 6H) ; 1,66 (m, 2H) ; de 2,49 à 2,56 (m partiellement masqué, 6H) ; 3,26 (m, 2H) ; 3,82 (s, 3H) ; 6,97 (d large, J = 9,0 Hz, 2H); 7,20 (m étalé, 1H) ; 8,01 (d large, J = 9,0 Hz, 2H).

**<u>Exemple 19</u>**

**6-(1-méthyl-5-fluoro-indol-3-yl)-3-(3-diéthylaminopropylamino)-4*H*-1,2,4-triazin-5-**one (composé n°3)

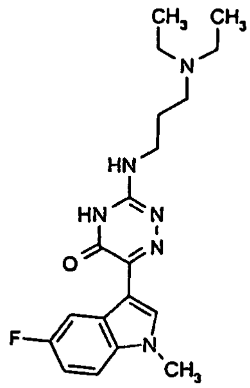

19.1 acide 5-fluoro-1*H*-indol-3-yl-oxo-acétique

A une solution maintenue entre 0°C et 5°C de 5-fluoroindole dans 10 $cm^3$ d'éther éthylique, est ajoutée à la seringue en 15 min une solution de 523 μl de chlorure d'oxalyle dans 5 $cm^3$ d'éther éthylique. Le milieu réactionnel, initialement incolore, se teinte en jaune puis un précipité jaune apparaît peu à peu. La suspension est agitée 1 h 30 à une température voisine de 20°C puis elle est refroidie dans un bain de glace et elle est alcalinisé avec de la soude 1 N. On agite jusqu'à dissolution complète du précipité. La solution est extraite deux fois à l'acétate d'éthyle. Les phases aqueuses sont rassemblées, acidifiées par de l'acide chlorhydrique concentré et refroidies dans un bain de glace. Le précipité est collecté par filtration, essoré et séché sous pression réduite (2,7 kPa) à 50°C pour donner 795 mg d'acide 5-fluoro-1*H*-indol-3-yl-oxo-acétique sous forme d'un solide jaune pâle.

19.2 acide (5-fluoro-1-méthyl-1*H*-indol-3-yl)-cxo-acétique

A une suspension refroidie dans un bain de glace de 622 mg d'acide 5-fluoro-1H-indol-3-yl-oxo-acétique, préparé à l'étape 19.1, dans 10 $cm^3$ d'acétone, est ajouté en une fois 1,01 g de potasse. On laisse remonter le mélange à une température voisine de 20°C, on l'agite quelques minutes à cette température, on le refroidit à nouveau à l'aide d'un bain de glace puis on introduit 757 μl d'iodure de méthyle. Après 15 h d'agitation à une température voisine de 20°C, la suspension incolore très épaisse est diluée par de l'acétone, additionnée de 373 μl d'iodure de méthyle puis agitée encore 1 h à une température voisine de 20°C. On ajoute de l'eau au milieu réactionnel et on agite jusqu'à dissolution complète. La solution est extraite deux fois à l'acétate d'éthyle et la phase organique est lavée à l'eau. Les phases aqueuses réunies sont acidifiées avec de l'acide chlorhydrique concentré et refroidies dans un bain de glace. Le précipité formé est collecté par filtration et séché sous pression réduite (2,7 kPa) à 50°C pendant 15 h pour donner 517 mg d' acide (5-fluoro-1-méthyl-1H-indoi-3-yl)-oxo-acétique sous la forme de paillettes jaune pâle.

19.3 6-(1-méthyl-5-fluoro-indol-3-yl)-3-(3-diéthylaminopropylamino)-4*H*-1,2,4-triazin-5-one

Un mélange de 630 mg d'iodohydrate de *N*-amino-*N'*-(3-diéthylamino-propyl)-guanidine, préparé à l'étape 12.1 de l'exemple 12, et de 442 mg d'acide (5-fluoro-1-méthyl-1*H*-indol-3-yl)-oxo-acétique, préparé à l'étape 19.2, dans 2 $cm^3$ d'eau est chauffé pendant 15 min à 140°C au four à micro-ondes puis refroidi à une température voisine de 20°C.

Le mélange orangé résultant est empâté à l'éthanol et filtré. Le solide jaune obtenu est séché sous pression réduite (2,7 kPa) à 30°C et il est purifié par une chromatographie sur silice [éluant : dichlorométhane / méthanol / ammoniaque 32% (80 / 20 / 1 en volumes)] pour donner 0,078 g de 6-(1-méthyl-5-fluoro-indol-3-yl)-3-(3-diéthylaminopropylamino)-4*H*-1,2,4-triazin-5-one sous la forme d'un solide blanc. Spectre de masse (ES) : m/z = 373 ($MH^+$), 300 ($M-C_4 H_{11}N+H^+$). Spectre RMN 1H (400MHz ) - δ en ppm - dans le DMSO-d6 : 1,01 (t, J = 7,0 Hz, 6H) ; 1,68 (m large, 2H) ; de 2,43 à 2,58 (m, partiellement masqués, 6H) ; 3,29 (m, 2H) ; 3,88 (s, 3H) ; de 7,09 à 7,20 (m, 2H) ; 7,54 (dd, J = 5,5 et 8,0 Hz, 1H); 8,02 (dd, J = 2,0 et 10,5 Hz, 1H) ; 8,68 (s, 1H).

**Exemple 20**

**6-(1-méthyl-6-fluoro-indol-3-yl)-3-(3-diéthylaminopropylamino)-4*H*-1,2,4-triazin-5-one** (composé n°4)

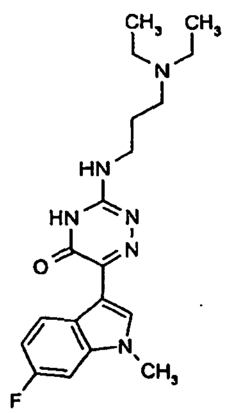

20.1 acide 6-fluoro-1*H*-indol-3-yl-oxo-acétique

A une solution maintenue entre 0°C et 5°C de 1,0 g de 6-fluoroindole dans 15 $cm^3$ d'éther éthylique, est additionnée à la seringue, en 15 min, une solution de 775 μl de chlorure d'oxalyle dans 7 $cm^3$. Le milieu réactionnel, initialement incolore, se teinte en jaune puis un précipité jaune se forme peu à peu. La suspension est agitée 2 h 30 à une température voisine de 20°C, refroidie dans un bain de glace et alcalinisée avec de la soude 1 N. On agite le mélange jusqu'à dissolution complète du précipité. La solution est extraite deux fois à l'acétate d'éthyle. Les phases aqueuses sont rassemblées, acidifiées avec de l'acide chlorhydrique concentré et refroidies dans un bain de glace. Le précipité formé est collecté par filtration puis séché sous pression réduite (2,7 kPa) à 50°C pour donner 1,12 g d'acide 6-fluoro-1*H*-indol-3-yl-oxo-acétique sous forme d'un solide jaune vif.

20.2 acide (6-fluoro-1-méthyl-1*H*-indol-3-yl)-oxo-acétique

A une suspension refroidie dans un bain de glace de 1,036 g d'acide 6-fluoro-1*H*-indol-3-yl-oxo-acétique, préparé à l'étape 20.1, dans 17 $cm^3$ d'acétone, est ajouté en une fois 1,684 g de potasse. On laisse remonter le mélange à une température voisine de 20°C et on l'agite quelques minutes à cette température. Après avoir à nouveau refroidi le milieu réactionnel à l'aide d'un bain de glace, on lui ajoute 1,245 $cm^3$ d'iodure de méthyle, laisse revenir le mélange à une température voisine de 20°C et agite 15 h à cette température. La suspension résultante, très épaisse, est additionnée de 650 μl d'iodométhane, agitée 24 h supplémentaires à une température voisine de 20°C, chauffée 30 min au reflux, laissée refroidir à une température voisine de 20°C et additionnée d'eau. On agite jusqu'à dissolution complète puis extrait le mélange deux fois à l'acétate d'éthyle. La phase aqueuse est lavée à l'eau. Les phases aqueuses réunies sont acidifiées avec de l'acide chlorhydrique concentré et refroidies dans un bain de glace. Le précipité est essoré et séché sous pression réduite (2,7 kPa) à 50°C pendant 15 h pour donner 1,04 g d'acide (6-fluoro-1-méthyl-1*H*-indol-3-yl)-oxo-acétique.

20.3 6-(1-méthyl-6-fluoro-indol-3-yl)-3-(3-diéthylaminopropylamino)-4*H*-1,2,4-triazin-5-one

Un mélange de 630 mg d'iodohydrate de *N*-amino-*N*'-(3-diéthylamino-propyl)-guanidine, préparé à l'étape 12.1 de l'exemple 12, et de 442 mg d'acide (6-fluoro-1-méthyl-1*H*-indol-3-yl)-oxo-acétique, préparé à l'étape 20.2, dans 2

$cm^3$ d'eau est chauffé pendant 15 min à 140°C au four micro-ondes puis il est refroidi à une température voisine de 20°C. Le mélange orangé obtenu est empâté à l'éthanol et le solide résultant est essoré et séché sous pression réduite (2,7 kPa) à 40°C puis ce solide est purifié par une chromatographie sur silice [éluant : dichlorométhane / méthanol / ammoniaque 32% (90 / 10 / 1 en volumes)] pour donner 0,324 g de 6-(1-méthyl-6-fluoro-indol-3-yl)-3-(3-diéthylamino-propylamino)-4*H*-1,2,4-triazin-5-one sous la forme d'un solide jaune pâle. Spectre de masse (ES) : m/z = 373 ($MH^+$) ; Spectre RMN 1H (300MHz ) - δ en ppm - dans le DMSO-d6 : 1,09 (m large, 6H) ; 1,77 (m large, 2H) ; de 2,40 à 2,86 (m, partiellement masqué, 6H) ; 3,30 (m masqué, 2H) ; 3,84 (s, 3H) ; de 6,98 à 7,14 (m, 2H) ; 7,40 (dd, J = 2,5 et 11,0 Hz, 1H) ; 8,29 (dd, J = 5,0 et 9,0 Hz, 1H) ; 8,62 (s, 1H).

**Exemple 21**

**6-(3-méthoxyphényl)-3-(3-diéthylaminopropylamino)-4*H*-1,2,4-triazin-5-one** (composé n°16)

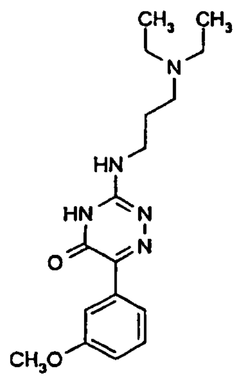

21.1 3-méthoxy-phényl-oxo-acétate d'éthyle

Un mélange, maintenu à une température comprise entre à -78°C et -65°C, de 1,5 $cm^3$ d'oxalate de diéthyle dans 10 $cm^3$ d'éther, est additionné de 10 $cm^3$ d'une solution 1 N de bromure de 3-méthoxyphénylmagnesium dans le tétrahydrofuranne. En fin d'addition, on laisse remonter lentement la température à 10°C et neutralise le milieu à l'aide d'une solution saturée de chlorure d'ammonium. Le mélange est extrait deux fois à l'éther éthylique. Les phases organiques réunies sont lavées à l'eau, séchées sur sulfate de sodium, filtrées et concentrées à sec sous pression réduite (2,7 kPa). On isole 2,15 g de 3-méthoxy-phényl-oxo-acétate d'éthyle en mélange avec environ 30 % de d'oxalate de diéthyle n'ayant pas réagi sous forme d'une huile jaune clair.

21.2 acide 3-méthoxy-phényl-oxo-acétique

Un mélange de 2,14 g de 3-méthoxy-phényl-oxo-acétate d'éthyle, préparé à l'étape 21.1, et de 6 $cm^3$ de soude 2 N est agité pendant 30 min à une température voisine de 20°C puis il est additionné d'environ 15 $cm^3$ d'eau ce qui permet de dissoudre l'insoluble, et agité encore 30 min à une température voisine de 20°C. Le mélange réactionnel est extrait à l'acétate d'éthyle et la phase organique est lavée deux fois à l'eau. Les phases aqueuses sont réunies acidifiées par de l'acide chlorhydrique concentré en présence d'acétate d'éthyle et extraites deux fois à l'acétate d'éthyle. Les phases organiques sont réunies, lavées deux fois à l'eau, séchées sur sulfate de sodium anhydre, filtrées puis concentrées à sec sous pression réduite (2,7 kPa). On obtient une huile jaune qui est séchée à la pompe à palette donner 1,34 g d'acide 3-méthoxy-phényl-oxo-acétique.

21.3 6-(3-méthoxyphényl)-3-(3-diéthylaminopropylamino)-4*H*-1,2,4-triazin-5-one

Un mélange de 630 mg d'iodohydrate de N-amino-N'-(3-diéthylamino-propyl)guanidine, préparé à l'étape 12.1 de l'exemple 12, et de 360 mg d'acide 3-méthoxy-phényl-oxo-acétique dans 2 $cm^3$ d'eau est chauffé pendant 15 min à 140°C au four à micro-ondes puis refroidi à une température voisine de 20°C. Le milieu réactionnel est évaporé à sec sous pression réduite (2,7 kPa) et le résidu est purifié par une chromatographie sur silice [éluant : dichlorométhane / méthanol / ammoniaque 32% (90 / 10 / 1 en volumes)] pour donner, après empâtage à l'acétate d'éthyle et essorage, 0,271 g de 6-(3-méthoxyphényl)-3-(3-diéthylaminopropylamino)-4*H*-1,2,4-triazin-5-one sous la forme d'un solide blanc.

Spectre de masse (ES) : m/z = 332 ($MH^+$) ; Spectre RMN 1H (400MHz ) - δ en ppm - dans le DMSO-d6 : 1,02 (t, J = 7,5 Hz, 6H) ; 1,70 (m, 2H) ; de 2,45 à 2,66 (m partiellement masqué, 6H); 3,30 (m partiellement masqué, 2H) ; 3,80 (s, 3H) ; 7,00 (m, 1H) ; de 7,25 à 7,36 (m, 2H) ; 7,59 (m, 2H).

**Exemple 22**

**6-(4-(4-méthylphényl))-3-(3-diéthylaminopropylamino)-4*H*-1,2,4-triazin-5-one** (composé n°19)

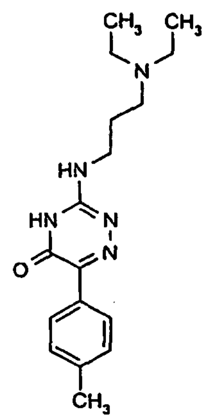

Un mélange de 630 mg d'iodohydrate de *N*-amino-*N*'-(3-diéthylamino-propyl)-guanidine, préparé à l'étape 12.1 de l'exemple 12, et de 328 mg d'acide 2-(4-méthylphényl)-2-oxo-acétique dans 2 $cm^3$ d'eau est chauffé pendant 15 min à 140°C au four à micro-ondes puis refroidi à une température voisine de 20°C. Le milieu réactionnel est évaporé à sec sous pression réduite (2,7 kPa) et le résidu est purifié par une chromatographie sur silice [éluant : dichlorométhane / méthanol / ammoniaque 32% (85 / 15 / 1 en volumes)] pour donner, après empâtage à l'éther éthylique et essorage, 0,467g de 6-(4-(4-méthylphényl))-3-(3-diéthylaminopropylamino)-4*H*-1,2,4-triazin-5-one sous forme d'un solide jaune pâle. Spectre de masse (ES) : m/z = 316 ($MH^+$), 243 ($M-C_4H_{11}N+H^+$) ; Spectre RMN 1H (400MHz ) - δ en ppm - dans le DMSO-d6 : 1,21 (t, J = 7,5 Hz, 6H) ; 1,89 (m, 2H) ; 2,35 (s, 3H) ; de 3,05 à 3,20 (m, 6H) ; 3,32 (m masqués, 2H) ; de 6,90 à 7,35 (m très étalé, 5H) ; 7,90 (d large, J = 9,0 Hz, 2H).

**Exemple 23**

**iodohydrate de 6-(1-méthyl-4-fluor-indol-3-yl)-3-(3-diéthylaminopropylamino)-4*H*-1,2,4-triazin-5-one** (composé n°5)

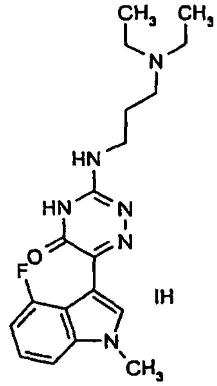

23.1 acide 4-fluoro-1*H*-indol-3-yl-oxo-acétique

A une solution maintenue à une température comprise entre 0˚C et 5˚C de 1 g de 6-fluoroindole dans 15 $cm^3$ d'éther éthylique, est additionnée à la seringue, en 15 min, une solution de 800 µl de chlorure d'oxalyle dans 7 $cm^3$ d'éther. Dans le milieu réactionnel, des précipités jaunes se forment. La suspension est agitée 3 h à une température voisine de 20˚C, refroidie dans un bain de glace et est alcalinisée avec de la soude 1 N. On agite jusqu'à dissolution complète du précipité. La solution est extraite deux fois à l'acétate d'éthyle. Les phases aqueuses sont rassemblées, acidifiées avec de l'acide chlorhydrique concentré et refroidies dans un bain de glace. Le précipite formé est essoré puis séché sous pression réduite (2,7 kPa) à 50˚C pour donner 892 mg d'acide 4-fluoro-1*H*-indol-3-yl-oxo-acétique sous la forme d'un solide jaune vif.

23.2 acide (4-fluoro-1-méthyl-1*H*-indol-3-yl)-oxo-acétique

Une suspension refroidie dans un bain de glace de 888 mg d'acide 4-fluoro-1*H*-indol-3-yl-oxo-acétique, préparé à l'étape 23.1, dans 15 $cm^3$ d'acétone, est additionnée en une fois de 1,443 g de potasse. On laisse remonter à une température voisine de 20˚C, agite quelques minutes à cette température, refroidit à nouveau à l'aide d'un bain de glace puis introduit 1,067 $cm^3$ d'iodure de méthyle. Après 60 h d'agitation à une température voisine de 20˚C, on ajoute 550 µl d'iodométhane à la suspension très épaisse obtenue et laisse le milieu sous agitation pendant 9 h supplémentaires à une température voisine de 20˚C. Le mélange réactionnel est extrait deux fois à l'acétate d'éthyle et les phases organiques sont lavées à l'eau. Les phases aqueuses réunies sont acidifiées avec de l'acide chlorhydrique concentré et refroidies dans un bain de glace. Le précipité est essoré et séché sous pression réduite (2,7 kPa) à 50˚C pendant 15 h pour donner 913 mg d'acide (4-fluoro-1-méthyl-1*H*-indol-3-yl)-oxo-acétique sous la forme d'un solide brun-jaune.

23.3 iodohydrate de 6-(1-méthyl-4-fluor-indol-3-yl)-3-(3-diéthylaminopropylamino)-4*H*-1,2,4-triazin-5-one

Un mélange de 630 mg d'iodohydrate de *N*-amino-*N*'-(3-diéthylamino-propyl)-guanidine, préparé à l'étape 12.1 de l'exemple 12, et de 442 mg d'acide (4-fluoro-1-méthyl-1*H*-indol-3-yl)-oxo-acétique, préparé à l'étape 23.2, dans 2 $cm^3$ d'eau est chauffé pendant 15 min à 140˚C au four à micro-ondes puis refroidi à une température voisine de 20˚C. Le milieu réactionnel est évaporé à sec sous pression réduite (2,7 kPa) et le résidu obtenu est purifié par chromatographie sur silice [éluant : dichlorométhane / méthanol / ammoniaque 32% (85 / 15 / 1 en volumes)] pour donner, après empâtage au méthanol et essorage, 0,150 g d'iodohydrate de 6-(1-méthyl-4-fluor-indol-3-yl)-3-(3-diéthylaminopropylamino)-4*H*-1,2,4-triazin-5-one sous forme de solide blanc. Spectre de masse (ES) : m /z = 373 ($MH^+$) ; Spectre RMN 1H (300MHz ) - δ en ppm - dans le DMSO-d6 : 1,21 (t, J = 7,5 Hz, 6H) ; 1,90 (m, 2H) ; 3,14 (m, 6H) ; 3,35 (m partiellement masqué, 2H) ; 3,87 (s, 3H) ; 6,87 (dd, J = 5,5 et 11,0 Hz, 1H) ; de 7,05 à 7,37 (m, 3H) ; 8,18 (s, 1H) ; de 9,00 à 9,25 (m très étalé, 1H) ; 12,4 (m étalé, 1H).

Le tableau qui suit illustre les structures chimiques et les propriétés physiques de quelques exemples de composés selon l'invention. Dans ce tableau :

- dans la colonne « sel », « - » représente un composé sous forme de base libre, alors que « HCl », « HCl, $H_2O$ » et « HI » représentent respectivement un composé sous forme de chlorhydrate, de chlorhydrate hydrate et de iodohydrate, et le rapport entre parenthèses est le rapport (acide:base),
- Et représente un groupe éthyle.

Tableau

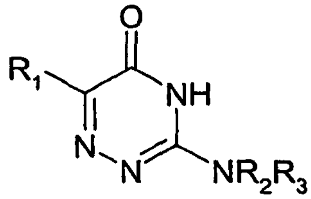

(I)

(suite)

| N° | $R_1$ | $R_2$ | $R_3$ | Sel |
|---|---|---|---|---|
| 1 | 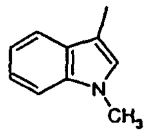 | H | $-(CH_2)_3N(Et)_2$ | - |
| 2 | 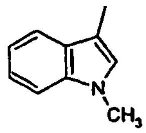 | H | 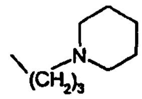 | - |
| 3 | 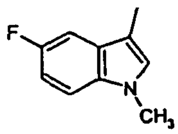 | H | $-(CH_2)_3N(Et)_2$ | - |
| 4 | 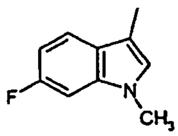 | H | $-(CH_2)_3N(Et)_2$ | - |
| 5 | 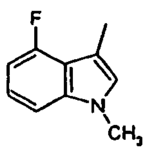 | H | $-(CH_2)_3N(Et)_2$ | HI (1/1) |
| 6 | 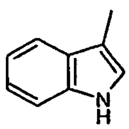 | H | 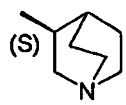 | HCl, $H_2O$ (1/1/1) |
| 7 | 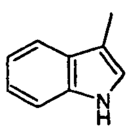 | H | $-(CH_2)_3N(Et)_2$ | - |
| 8 | 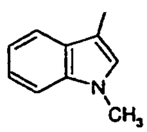 | H | 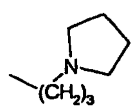 | - |
| 9 | 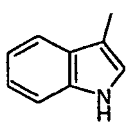 | H | 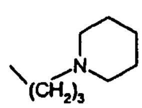 | - |
| 10 | 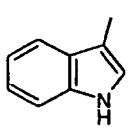 | H | 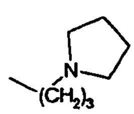 | - |

(suite)

| N° | $R_1$ | $R_2$ | $R_3$ | Sel |
|---|---|---|---|---|
| 11 | 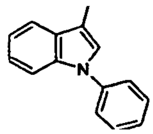 | H | $-(CH_2)_3N(Et)_2$ | - |
| 12 | 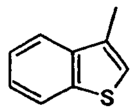 | H | $-(CH_2)_3N(Et)_2$ | - |
| 13 | 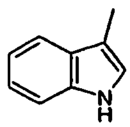 | H | 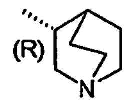 | HCl (1/1) |
| 14 | 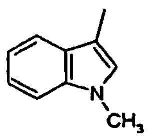 | H | $-(CH_2)_3N(CH_3)_2$ | - |
| 15 | 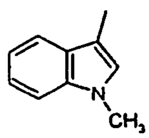 | H | 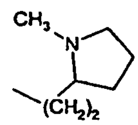 | - |
| 16 | 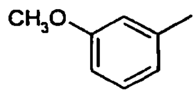 | H | $-(CH_2)_3N(Et)_2$ | - |
| 17 | 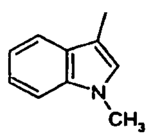 | H | $-(CH_2)_3N(Et)_2$ | - |
| 18 | 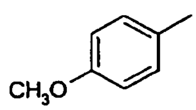 | H | $-(CH_2)_3N(Et)_2$ | - |
| 19 | 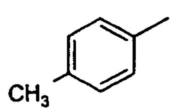 | H | $-(CH_2)_3N(Et)_2$ | - |
| 20 | 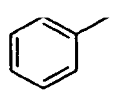 | H | $-(CH_2)_3N(Et)_2$ | - |

(suite)

| N° | $R_1$ | $R_2$ | $R_3$ | Sel |
|---|---|---|---|---|
| **21** | 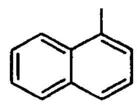 | H | $-(CH_2)_3N(Et)_2$ | - |
| **22** | 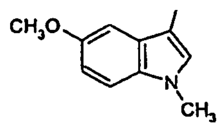 | H | $-(CH_2)_3N(Et)_2$ | HI (1/1) |
| **23** | 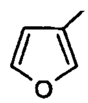 | H | $-(CH_2)_3N(Et)_2$ | - |

Les composés de l'invention ont été testés quant à leur capacité à lier les récepteurs nicotiniques contenant la sous-unité α7 à l'aide d'un test de liaison sur préparations de membranes de cerveau de rat selon les méthodes décrites ci-dessous:

Préparations des membranes

Des échantillons congelés d'hippocampe de cerveau de rats femelles Sprague-Dawley ont été conservés à -20°C jusqu'à utilisation. Les hippocampes de 10 rats ont été regroupés et homogénéisés à l'aide d'un broyeur de type Polytron dans 10 volumes d'un tampon refroidis dans la glace de composition suivante: KCl (11 mM); $KH_2PO_4$ (6mM); NaCl (137 mM); $Na_2HPO_4$ (8 mM); HEPES (20 mM); iodoacetamide (5 mM); EDTA (1,5 mM); PMSF (0,1 mM). Le pH a été ajusté à 7,4 à l'aide de NaOH. le mélange obtenu a été centrifugé à 24000 g pendant 20 minutes à 4°C et le culot remis en suspension dans 20 volumes d'eau glacée. Après 60 minutes d'incubation à 4°C, un nouveau culot a été obtenu par centrifugation à 24000 g pendant 20 minutes à 4°C. Ce dernier a été remis en suspension dans du tampon de composition ci-dessus et congelé à -20°C. Le jour de l'essai, les membranes ont été décongelées, centrifugées à 24000 g pendant 20 minutes, puis remises en suspension à une concentration finale de 2-5 mg de protéines/mL dans du tampon phosphate de Dulbecco à pH 7,4 contenant 0,05% d'albumine sérique de boeuf.

Mesure de l'affinité pour les récepteurs contenant la sous-unité α7

La liaison des composés de l'invention pour les récepteurs contenant la sous-unité α7 a été mesurée par compétition vis-à-vis de [$^3$H]-methyllycaconitine ([$^3$H]-MLA), un traceur radiomarqué reconnaissant les récepteurs au (Davies et al., Neuropharmacology 1999, 38, 679-690) selon des méthodes classiques adaptées au format de plaques 96-puits. La capacité des composés de l'invention à déplacer la liaison de [$^3$H]-MLA sur des membranes d'hippocampe de rat a été déterminée en duplicate après 2 heures d'incubation à une température voisine de 20°C. Chaque puits contenait un échantillon d'environ 150 μg de protéines membranaires, 5 nM de [$^3$H]-MLA et l'un des composés de l'invention dilué à une concentration déterminée dans du tampon phosphate de Dulbecco à pH 7,4 contenant 0,05% d'albumine sérique de boeuf pour un volume final de 150 μL. La liaison non-spécifique a été déterminée dans des puits spécifiques contenant 10 μM de MLA non-radiomarquée. L'incubation a été stoppée en filtrant le contenu de chaque puits à travers des filtres en fibres de verre (Whatman GF/B) préalablement trempés dans une solution de polyéthylènimine à 0,33% dans du tampon phosphate de Dulbecco pour diminuer la liaison non-spécifique. Les filtres ont ensuite été lavés 3 fois avec du tampon phosphate de Dulbecco, puis séchés à 50°C pendant environ 2 heures. La radioactivité retenue sur les filtres a été mesurée par application de scintillant (MeltiLex A, Perkin Elmer) suivi d'un comptage en luminométrie (Trilux 1450 microbeta, Perkin-Elmer).

Analyse des données

Pour chaque composé testé, la radioactivité résiduelle sur les filtres a été exprimée en coups par minutes. Les déterminations en duplicate ont été moyennées et la concentration de composé qui inhibe de moitié la liaison spécifique

du traceur radioactif ($CI_{50}$) a été calculée par régression curviligne à l'aide d'un logiciel spécifique (GraphPad Prism). Les constantes d'affinité apparente Ki des composés de l'invention ont été calculées à l'aide de l'équation de Cheng et Prusoff (Cheng et Prusoff, Biochem. Pharmacol. 1973, 22, 3099-3108).
Les composés de l'invention qui ont été étudiés dans ce test présentent une valeur de Ki inférieure à 10 $\mu$M. Par exemple, les composés n° 11 et 12, ont montré une valeur de Ki de respectivement 0,737 et 0,957 $\mu$M.

Il apparaît donc que les composés selon l'invention sont des ligands des récepteurs nicotiniques contenant la sous-unité $\alpha$7.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement curatif et/ou symptomatique, pour la prévention, le diagnostic et/ou le suivi de l'évolution des troubles ou maladies impliquant une perturbation du fonctionnement des récepteurs nicotiniques $\alpha$7 ou répondant favorablement à une modulation de ceux-ci.

Plus particulièrement, les composés de l'invention peuvent être utiles dans des troubles ou maladies psychiatriques ou neurologiques du système nerveux central impliquant des altérations des fonctions cognitives, de l'attention, des facultés de concentration, des capacités d'apprentissage et de mémorisation, ou du traitement de l'information sensorielle.

Ils peuvent également être utiles dans le traitement, la prévention, le diagnostic et/ou le suivi de l'évolution de maladies impliquant des processus neurodégénératifs spontanés ou consécutifs à des lésions, des douleurs aiguës ou chroniques et de maladies impliquant des phénomènes inflammatoires.

Les composés de l'invention peuvent être utiles dans le cas de troubles ou de maladies psychiatriques ou neurologiques du système nerveux central tels que par exemple les troubles cognitifs, les déficits mnésiques liés à l'âge ou à des infections virales ou bactériennes, les altérations des facultés d'apprentissage, de concentration et de mémorisation, les altérations cognitives légères, les démences séniles, les démences vasculaires, les démences à corps de Levy, la maladie d'Alzheimer, la maladie de Parkinson, la chorée de Huntington, le syndrome de Gilles de la Tourette, les dégénérescences neuronales consécutives à un traumatisme, aux accidents vasculaires cérébraux, à l'ischémie ou à l'hypoxie cérébrale, l'atrophie multisystématisée, la paralysie supranucléaire progressive, la sclérose latérale amyotrophique, les neuropathies périphériques, les troubles moteurs tels que les dyskinésies, les dyskinésies tardives, les hyperkinésies, les dystonies et les épilepsies, les déficits de l'attention liés à l'hyperactivité, la schizophrénie, la dépression, la psychose maniaco-dépressive, l'anxieté, les phobies, les troubles obsessifs compulsifs, le syndrome de stress post-traumatique, les attaques de panique, les troubles de l'alimentation tels que l'anorexie, la boulimie et l'obésité, les troubles du sommeil y compris ceux associés aux décalages horaires. Les composés de l'invention peuvent être utiles pour instaurer une réduction de la consommation de substances addictives, pour aider au maintien de l'abstinence vis à vis de celles-ci ou pour en atténuer les symptômes du sevrage. Dans le cadre de la présente invention, le terme de « substance addictive » s'applique à des substances licites ou illicites dont la consommation peut donner lieu à abus et/ou dépendance telles que par exemple la nicotine et les produits du tabac, l'alcool, les dérivés du cannabis, les opiacés, la cocaine, les barbituriques, les benzodiazépines et les psychostimulants.

Les composés de l'invention peuvent également présenter un intérêt dans le traitement des douleurs aiguës ou chroniques telles que les douleurs post-chirurgicales, les douleurs consécutives à une amputation (douleur du membre fantôme), les douleurs associées aux lésions cancéreuses, aux migraines, aux neuropathies et les douleurs musculaires telles que les fibromyalgies. De plus, les composés de l'invention peuvent être utilisés dans le cadre du traitement de troubles ou maladies impliquant des processus inflammatoires tels que par exemple, au niveau du tractus gastro-intestinal : la colite ulcéreuse, la maladie de Crohn, le syndrome du colon irritable, les diarrhées, et ailleurs dans l'organisme les arthrites (y-compris l'arthrite rhumatoide) et les inflammations cutanées telles que l'acnée. Enfin, les composés de l'invention peuvent être utiles dans les désordres endocriniens tels que le phéochromocytome et les troubles associés à la contraction des muscles lisses. Dans le cadre de la présente invention, le terme « composé-traceur » désigne les composés de l'invention, leurs énantiomères ou leurs pro-drogues utilisés ou non sous une forme marquée permettant leur détection par des moyens physiques tels que décrits plus haut. Le marquage consiste en le remplacement d'un ou plusieurs atomes dans les composés de l'invention par un isotope de masse atomique ou de nombre de masse différent de la masse atomique ou du nombre de masse de ces atomes tels qu'ils sont habituellement rencontrés dans la nature. Il peut également consister en l'adjonction aux composés de l'invention de groupements chimiques porteurs de tels isotopes à l'aide par exemple de réactifs de méthylation. Les isotopes utilisés peuvent être par exemple des radionuclides isotopes de l'hydrogène, du carbone, de l'azote, de l'oxygène, du fluor, du phosphore, du soufre, du chlore, de l'iode ou du technétium tels que respectivement $^{2}H$, $^{3}H$, $^{11}C$, $^{13}C$, $^{14}C$, $^{13}N$, $^{15}O$, $^{17}O$, $^{18}F$, $^{35}S$, $^{38}Cl$, $^{123}I$, $^{125}I$, $^{131}I$. Les composés marqués peuvent être synthétisés selon les méthodes décrites dans les modes opératoires de la présente invention en substituant un ou plusieurs réactifs dans le processus de synthèse par des réactifs identiques contenant le ou les isotopes marqueurs.

Les composés selon l'invention peuvent donc être utilisés pour la préparation de médicaments, en particulier

de médicaments destinés au traitement à la prévention, au diagnostic et/ou au suivi de l'évolution des troubles ou maladies ci-dessus mentionnés.

Selon un autre de ses aspects, la présente invention concerne des compositions pharmaceutiques comprenant, en tant que principe actif, un composé selon l'invention.

Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

## Revendications

1. Composé répondant à la formule (I)

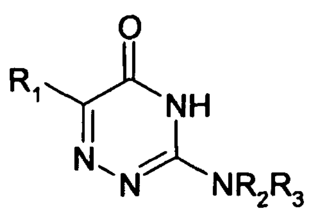

(I)

dans laquelle

$R_1$ représente un groupe hétéroaryle ou aryle, ledits groupes hétéroaryle ou aryle étant éventuellement substitués par un ou plusieurs groupes choisis parmi les atomes d'halogène et les groupes $(C_1-C_4)$alkyle, $(C_1-C_4)$alcoxy, $(C_3-C_7)$cycloalkyle, aryle, hydroxy, cyano, $-NH_2$, $-NO_2$ ;
$R_2$ représente un atome d'hydrogène ou un groupe $(C_1-C_4)$alkyle ;
$R_3$ représente

- un groupe $-(CH_2)n-NR_4R_5$ dans lequel

n est égal à 2, 3 ou 4 et
$R_4$ et $R_5$ représentent, indépendamment l'un de l'autre, un groupe ($C_1$-$C_4$)alkyle, ou ($C_3$-$C_7$)cycloalkyle, ou bien $R_4$ et $R_5$ forment ensemble, avec l'atome d'azote qui les porte, un groupe ($C_3$-$C_9$) hétérocycloalkyle ; ou

- un groupe -($CH_2$)m$R_6$ dans lequel

m est égal à 0, 1, 2, 3 ou 4 et
$R_6$ représente un groupe un groupe ($C_3$-$C_9$)hétérocycloalkyle comportant au moins un atome d'azote et lié au noyau triazine par un atome de carbone, le groupe ($C_3$-$C_9$)hétérocycloalkyle étant éventuellement substitué par un ou plusieurs groupes ($C_1$-$C_4$)alkyle ;

ou bien $R_2$ et $R_3$ forment ensemble, avec l'atome d'azote qui les porte, un ($C_5$-$C_9$)hétérocycloalkyle comprenant 2 atomes d'azote ;
avec la condition que quand $R_1$ représente un phényle alors $R_4$ et $R_5$ ne représentent pas simultanément un méthyle ;
à l'état de base ou de sel d'addition à un acide, ainsi qu'à l'état d'hydrate ou de solvat.

**2.** Composé de formule (I) selon la revendication 1, **caractérisé en ce que** $R_1$ représente un groupe hétéroaryle, ledit groupe hétéroaryle étant éventuellement substitué par un ou plusieurs groupes, choisis parmi les atomes d'halogène et les groupes ($C_1$-$C_4$)alkyle, ($C_1$-$C_4$)alcoxy, ($C_3$-$C_7$)cycloalkyle, aryle, hydroxy, cyano, -$NH_2$, -$NO_2$;
à l'état de base ou de sel d'addition à un acide, ainsi qu'à l'état d'hydrate ou de solvat.

**3.** Composé de formule (I) selon la revendication 1, **caractérisé en ce que** $R_1$ représente un groupe hétéroaryle ou aryle, ledits groupes hétéroaryle ou aryle étant éventuellement substitués par un ou plusieurs groupes choisis parmi les atomes d'halogène et les groupes ($C_1$-$C_4$)alkyle, ($C_1$-$C_4$)alcoxy, aryle ;
à l'état de base ou de sel d'addition à un acide, ainsi qu'à l'état d'hydrate ou de solvat.

**4.** Composé de formule (I) selon la revendication 1, **caractérisé en ce que** $R_1$ représente un groupe hétéroaryle, ledit groupe hétéroaryle étant éventuellement substitué par un ou plusieurs groupes choisis parmi les atomes d'halogène et les groupes ($C_1$-$C_4$)alkyle, ($C_1$-$C_4$)alcoxy, aryle ;
à l'état de base ou de sel d'addition à un acide, ainsi qu'à l'état d'hydrate ou de solvat.

**5.** Composé de formule (I) selon l'une quelconque des revendications 1 à 4, **caractérisé en ce que** $R_2$ représente un atome d'hydrogène ;
à l'état de base ou de sel d'addition à un acide, ainsi qu'à l'état d'hydrate ou de solvat.

**6.** Composé de formule (I) selon l'une quelconque des revendications 1 à 5, **caractérisé en ce que**
$R_3$ représente

- un groupe -($CH_2$)n-$NR_4R_5$ dans lequel

n est égal à 2, 3 ou 4 et
$R_4$ et $R_5$ représentent, indépendamment l'un de l'autre, un groupe ($C_1$-$C_4$)alkyle, ou bien $R_4$ et $R_5$ forment ensemble, avec l'atome d'azote qui les porte, un groupe ($C_3$-$C_7$)hétérocycloalkyle ; ou

- un groupe -($CH_2$)m$R_6$ dans lequel

m est égal à 0, 1, 2, 3 ou 4 et
$R_6$ représente un groupe ($C_3$-$C_7$)hétérocycloalkyle comportant au moins un atome d'azote et lié au noyau triazine par un atome de carbone, le groupe ($C_3$-$C_7$)hétérocycloalkyle étant éventuellement substitué par un ou plusieurs groupes ($C_1$-$C_4$)alkyle ;

avec la condition que quand $R_1$ représente un phényle alors $R_4$ et $R_5$ ne représentent pas simultanément un méthyle ;
à l'état de base ou de sel d'addition à un acide, ainsi qu'à l'état d'hydrate ou de solvat.

**7.** Composé de formule (I) selon l'une quelconque des revendications 1 à 6, **caractérisé en ce que**
$R_1$ représente un groupe indolyle, ledit groupe indolyle étant éventuellement substitué par un ou plusieurs groupes choisis parmi les atomes d'halogène et les groupes ($C_1$-$C_4$)alkyle, ($C_1$-$C_4$)alcoxy, ($C_3$-$C_7$)cycloalkyle, aryle, -$NH_2$,

$-NO_2$;
$R_2$ représente un atome d'hydrogène ;
$R_3$ représente

- un groupe $-(CH_2)n-NR_4R_5$ dans lequel

  n est égal à 2, 3 ou 4 et
  $R_4$ et $R_5$ représentent, indépendamment l'un de l'autre, un groupe ($C_1$-$C_4$)alkyle, ou bien $R_4$ et $R_5$ forment ensemble, avec l'atome d'azote qui les porte, un groupe ($C_3$-$C_7$)hétérocycloalkyle ; ou

- un groupe $-(CH_2)mR_6$ dans lequel

  m est égal à 0, 1, 2, 3 ou 4 et

$R_6$ représente un groupe ($C_3$-$C_7$)hétérocycloalkyle, le groupe ($C_3$-$C_7$)hétérocycloalkyle étant éventuellement substitué par un ou plusieurs groupes ($C_1$-$C_4$)alkyle ;
à l'état de base ou de sel d'addition à un acide, ainsi qu'à l'état d'hydrate ou de solvat.

**8.** Composé de formule (I) selon l'une quelconque des revendications 1 à 7, **caractérisé en ce qu'**il est choisi parmi :

- 6-(1-méthyl-1*H*-indol-3-yl)-3-(3-pipéridin-1-yl-propylamino)-4*H*-1,2,4-triazin-5-one
- 3-(3-diéthylamino-propylamino)-6-(1-méthyl-1*H*-indol-3-yl)-4*H*-1,2,4-triazin-5-one
- 3-(3-diéthylamino-propylamino)-6-phényl-4*H*-1,2,4-triazin-5-one
- 3-(2-diéthylamino-éthylamino)-6-(1-méthyl-1*H*-indol-3-yl)-4*H*-1,2,4-triazin-5-one
- 3-(3-diméthylamino-propylamino)-6-(1-méthyl-1*H*-indol-3-yl)-4*H*-1,2,4-triazin-5-one
- 6-(1-méthyl-1*H*-indol-3-yl)-3-(3-pyrrolidin-1-yl-propylamino)-4*H*-1,2,4-triazin-5-one
- 6-(1-méthyl-1*H*-indol-3-yl)-3-[2-(1-méthyl-pyrrolidin-2-yl)-éthylamino]-4*H*-1,2,4-triazin-5-one
- 6-(1*H*-indol-3-yl)-3-(3-pyrrolidin-1-yl-propylamino)-4*H*-1,2,4-triazin-5-one
- 6-(1*H*-indol-3-yl)-3-(3-pipéridin-1-yl-propylamino)-4*H*-1,2,4-triazin-5-one
- 3-[(*S*)-(1-aza-bicyclo[2.2.2]oct-3-yl)amino]-6-(1*H*-indol-3-yl)-4*H*-1,2,4-triazin-5-one
- 3-[(*R*)-(1-aza-bicyclo[2.2.2]oct-3-yl)amino]-6-(1*H*-indol-3-yl)-4H-1,2,4-triazin-5-one)
- 6-(furan-3-yl)-3-(3-diéthylaminopropylamino)-4*H*-1,2,4-triazin-5-one)
- 6-(napht-1-yl)-3-(3-diéthylaminopropylamino)-4*H*-1,2,4-triazin-5-one
- 6-(1-méthyl-5-méthoxy-indol-3-yl)-3-(3-diéthylaminopropylamino)-4*H*-1,2,4-triazin-5-one
- 6-(1-phényl-indol-3-yl)-3-(3-diéthylaminopropylamino)-4*H*-1,2,4-triazin-5-one
- 6-(indol-3-yl)-3-(3-diéthylaminopropylamino)-4*H*-1,2,4-triazin-5-one
- 6-(benzothiophen-3-yl)-3-(3-diéthylaminopropylamino)-4*H*-1,2,4-triazin-5-one
- 6-(4-méthoxyphényl)-3-(3-diéthylaminopropylamino)-4*H*-1,2,4-triazin-5-one
- 6-(1-méthyl-5-fluoro-indol-3-yl)-3-(3-diéthylaminopropylamino)-4*H*-1,2,4-triazin-5-one
- 6-(1-méthyl-6-fluoro-indol-3-yl)-3-(3-diéthylaminopropylamino)-4*H*-1,2,4-triazin-5-one
- 6-(3-méthoxyphenyl)-3-(3-diéthylaminopropylamino)-4*H*-1,2,4-triazin-5-one
- 6-(4-(4-méthylphényl))-3-(3-diéthylaminopropylamino)-4*H*-1,2,4-triazin-5-one
- 6-(1-méthyl-4-fluor-indol-3-yl)-3-(3-diéthylaminopropylamino)-4*H*-1,2,4-triazin-5-one.

**9.** Procédé de préparation d'un composé de formule (I) selon l'une quelconque des revendications 1 à 8, **caractérisé en ce que** l'on fait réagir le composé de formule générale (V)

(V)

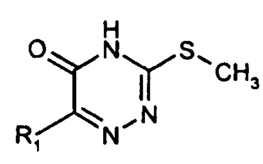

(V)

dans laquelle $R_1$ est tel que défini dans la formule générale (I) selon la revendication 1. avec une amine de formule générale $HNR_2R_3$ (VI) dans laquelle $R_2$ et $R_3$ sont tels que définis dans la formule générale (I) selon la revendication 1.

**10.** Procédé de préparation d'un composé de formule (I) selon l'une quelconque des revendications 1 à 8, **caractérisé en ce que** l'on fait réagir le composé de formule générale (VII),

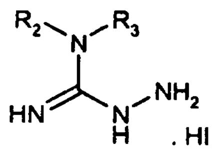

(VII)

dans laquelle $R_2$ et $R_3$ sont tels que définis dans la formule générale (I) selon la revendication 1, avec le composé de formule générale (II),

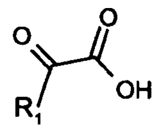

(II)

dans laquelle $R_1$ est tel que défini dans la formule générale (I).

**11.** Médicament, **caractérisé en ce qu'**il comprend un composé de formule (I) selon l'une quelconque des revendications 1 à 8, ou un sel d'addition de ce composé à un acide pharmaceutiquement acceptable, ou encore un hydrate ou un solvat du composé de formule (I).

**12.** Composition pharmaceutique, **caractérisée en ce qu'**elle comprend un composé de formule (I) selon l'une quelconque des revendications 1 à 8, ou un sel pharmaceutiquement acceptable, un hydrate ou un solvat de ce composé, ainsi qu'au moins un excipient pharmaceutiquement acceptable.

**13.** Composé de formule (I) selon l'une quelconque des revendications 1 à 8 pour la préparation d'un médicament destiné au traitement à la prévention, au diagnostic et/ou au suivi de l'évolution des troubles ou maladies psychiatriques ou neurologiques du système nerveux central impliquant des altérations des fonctions cognitives, de l'attention, des facultés de concentration, des capacités d'apprentissage et de mémorisation, ou du traitement de l'information sensorielle, de maladies impliquant des processus neurodégénératifs spontanés ou consécutifs à des lésions.

**14.** Composé de formule (I) selon l'une quelconque des revendications 1 à 8, pour la préparation d'un médicament destiné au traitement à la prévention, au diagnostic et/ou au suivi de l'évolution des douleurs aiguës ou chroniques.

**15.** Composé de formule (I) selon l'une quelconque des revendications 1 à 8 pour la préparation d'un médicament destiné au traitement à la prévention, au diagnostic et/ou au suivi de l'évolution de maladies impliquant des phénomènes inflammatoires

**Claims**

**1.** Compound corresponding to formula (I)

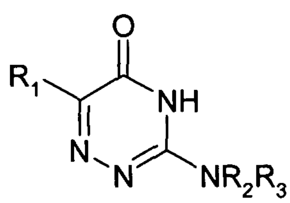

(I)

in which

$R_1$ represents a heteroaryl or aryl group, said heteroaryl or aryl groups being optionally substituted by one or

more groups selected from the halogen atoms and the $(C_1-C_4)$alkyl, $(C_1-C_4)$alkoxy, $(C_3-C_7)$cycloalkyl, aryl, hydroxy, cyano, $-NH_2$, $-NO_2$ groups;
$R_2$ represents a hydrogen atom or a $(C_1-C_4)$alkyl group;
$R_3$ represents

- a group $-(CH_2)n-NR_4R_5$ in which
n is equal to 2, 3 or 4 and
$R_4$ and $R_5$ represent, independently of one another, a $(C_1-C_4)$alkyl, or $(C_3-C_7)$cycloalkyl group, or alternatively $R_4$ and $R_5$ form together, with the nitrogen atom which carries them, a $(C_3-C_9)$heterocycloalkyl group; or
- a group $-(CH_2)mR_6$ in which
m is equal to 0, 1, 2, 3 or 4 and

$R_6$ represents a $(C_3-C_9)$heterocycloalkyl group containing at least one nitrogen atom and bound to the triazine ring by a carbon atom, the $(C_3-C_9)$heterocycloalkyl group being optionally substituted by one or more $(C_1-C_4)$ alkyl groups;
or alternatively $R_2$ and $R_3$ form together, with the nitrogen atom which carries them, a $(C_5-C_9)$heterocycloalkyl containing 2 nitrogen atoms;
with the condition that when $R_1$ represents a phenyl, $R_4$ and $R_5$ do not represent a methyl simultaneously;
in the form of base or of salt of addition to an acid, as well as in the form of hydrate or of solvate.

2. Compound of formula (I) according to Claim 1, **characterized in that** $R_1$ represents a heteroaryl group, said heteroaryl group being optionally substituted by one or more groups, selected from the halogen atoms and the $(C_1-C_4)$ alkyl, $(C_1-C_4)$alkoxy, $(C_3-C_7)$cycloalkyl, aryl, hydroxy, cyano, $-NH_2$, $-NO_2$ groups;
in the form of base or of salt of addition to an acid, as well as in the form of hydrate or of solvate.

3. Compound of formula (I) according to Claim 1, **characterized in that** $R_1$ represents a heteroaryl or aryl group, said heteroaryl or aryl groups being optionally substituted by one or more groups selected from the halogen atoms and the $(C_1-C_4)$alkyl, $(C_1-C_4)$alkoxy, aryl groups;
in the form of base or of salt of addition to an acid, as well as in the form of hydrate or of solvate.

4. Compound of formula (I) according to Claim 1, **characterized in that** $R_1$ represents a heteroaryl group, said heteroaryl group being optionally substituted by one or more groups selected from the halogen atoms and the $(C_1-C_4)$ alkyl, $(C_1-C_4)$alkoxy, aryl groups;
in the form of base or of salt of addition to an acid, as well as in the form of hydrate or of solvate.

5. Compound of formula (I) according to any one of Claims 1 to 4, **characterized in that** $R_2$ represents a hydrogen atom;
in the form of base or of salt of addition to an acid, as well as in the form of hydrate or of solvate.

6. Compound of formula (I) according to any one of Claims 1 to 5, **characterized in that**
$R_3$ represents

- a group $-(CH_2)n-NR_4R_5$ in which
n is equal to 2, 3 or 4 and
$R_4$ and $R_5$ represent, independently of one another, a $(C_1-C_4)$alkyl group, or alternatively $R_4$ and $R_5$ form together, with the nitrogen atom which carries them, a $(C_3-C_7)$heterocycloalkyl group; or
- a group $-(CH_2)mR_6$ in which
m is equal to 0, 1, 2, 3 or 4 and
$R_6$ represents a $(C_3-C_7)$heterocycloalkyl group containing at least one nitrogen atom and bound to the triazine ring by a carbon atom, the $(C_3-C_7)$heterocycloalkyl group being optionally substituted by one or more $(C_1-C_4)$ alkyl groups;

with the condition that when $R_1$ represents a phenyl, $R_4$ and $R_5$ do not represent a methyl simultaneously;
in the form of base or of salt of addition to an acid, as well as in the form of hydrate or of solvate.

7. Compound of formula (I) according to any one of Claims 1 to 6, **characterized in that**

$R_1$ represents an indolyl group, said indolyl group being optionally substituted by one or more groups selected from the halogen atoms and the $(C_1-C_4)$alkyl, $(C_1-C_4)$alkoxy, $(C_3-C_7)$cycloalkyl, aryl, $-NH_2$, $-NO_2$ groups;

$R_2$ represents a hydrogen atom;
$R_3$ represents

- a group $-(CH_2)n-NR_4R_5$ in which
n is equal to 2, 3 or 4 and
$R_4$ and $R_5$ represent, independently of one another, a $(C_1-C_4)$alkyl group, or alternatively $R_4$ and $R_5$ form together, with the nitrogen atom which carries them, a $(C_3-C_7)$heterocycloalkyl group; or
- a group $-(CH_2)mR_6$ in which
m is equal to 0, 1, 2, 3 or 4 and

$R_6$ represents a $(C_3-C_7)$heterocycloalkyl group, the $(C_3-C_7)$heterocycloalkyl group being optionally substituted by one or more $(C_1-C_4)$alkyl groups;
in the form of base or of salt of addition to an acid, as well as in the form of hydrate or of solvate.

**8.** Compound of formula (I) according to any one of Claims 1 to 7, **characterized in that** it is chosen from:

- 6-(1-methyl-1*H*-indol-3-yl)-3-(3-piperidin-1-yl-propylamino)-4*H*-1,2,4-triazin-5-one
- 3-(3-diethylamino-propylamino)-6-(1-methyl-1*H*-indol-3-yl)-4*H*-1,2,4-triazin-5-one
- 3-(3-diethylamino-propylamino)-6-phenyl-4*H*-1,2,4-triazin-5-one
- 3-(2-diethylamino-ethylamino)-6-(1-methyl-1*H*-indol-3-yl)-4*H*-1,2,4-triazin-5-one
- 3-(3-dimethylamino-propylamino)-6-(1-methyl-1*H*-indol-3-yl)-4*H*-1,2,4-triazin-5-one
- 6-(1-methyl-1*H*-indol-3-yl)-3-(3-pyrrolidin-1-yl-propylamino)-4*H*-1,2,4-triazin-5-one
- 6-(1-methyl-1*H*-indol-3-yl)-3-[2-(1-methyl-pyrrolidin-2-yl)-ethylamino]-4*H*-1,2,4-triazin-5-one
- 6-(1*H*-indol-3-yl)-3-(3-pyrrolidin-1-yl-propylamino)-4*H*-1,2,4-triazin-5-one
- 6-(1*H*-indol-3-yl)-3-(3-piperidin-1-yl-propylamino)-4*H*-1,2,4-triazin-5-one
- 3-[(*S*)-(1-aza-bicyclo[2.2.2]oct-3-yl)amino]-6-(1*H*-indol-3-yl)-4*H*-1,2,4-triazin-5-one
- 3-[(*R*)-(1-aza-bicyclo[2.2.2]oct-3-yl)amino]-6-(1*H*-indol-3-yl)-4*H*-1,2,4-triazin-5-one)
- 6-(furan-3-yl)-3-(3-diethylaminopropylamino)-4*H*-1,2,4-triazin-5-one)
- 6-(naphth-1-yl)-3-(3-diethylaminopropylamino)-4*H*-1,2,4-triazin-5-one
- 6-(1-methyl-5-methoxy-indol-3-yl)-3-(3-diethylaminopropylamino)-4*H*-1,2,4-triazin-5-one
- 6-(1-phenyl-indol-3-yl)-3-(3-diethylaminopropylamino)-4*H*-1,2,4-triazin-5-one
- 6-(indol-3-yl)-3-(3-diethylaminopropylamino)-4H-1,2,4-triazin-5-one
- 6-(benzothiophen-3-yl)-3-(3-diethylaminopropylamino)-4*H*-1,2,4-triazin-5-one
- 6-(4-methoxyphenyl)-3-(3-diethylaminopropylamino)-4*H*-1,2,4-triazin-5-one
- 6-(1-methyl-5-fluoro-indol-3-yl)-3-(3-diethylaminopropylamino)-4*H*-1,2,4-triazin-5-one
- 6-(1-methyl-6-fluoro-indol-3-yl)-3-(3-diethylaminopropylamino)-4*H*-1,2,4-triazin-5-one
- 6-(3-methoxyphenyl)-3-(3-diethylaminopropylamino)-4*H*-1,2,4-triazin-5-one
- 6-(4-(4-methylphenyl))-3-(3-diethylaminopropylamino)-4*H*-1,2,4-triazin-5-one
- 6-(1-methyl-4-fluoro-indol-3-yl)-3-(3-diethylamino-propylamino)-4*H*-1,2,4-triazin-5-one.

**9.** Method of preparation of a compound of formula (I) according to any one of Claims 1 to 8, **characterized in that** the compound of general formula (V)

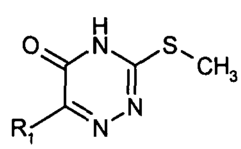
(V)

in which $R_1$ is as defined in general formula (I) according to Claim 1,
is reacted with an amine of general formula $HNR_2R_3$ (VI) in which $R_2$ and $R_3$ are as defined in general formula (I) according to Claim 1.

**10.** Method of preparation of a compound of formula (I) according to any one of Claims 1 to 8, **characterized in that** the compound of general formula (VII)

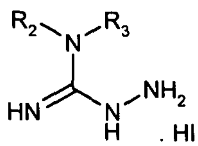

(VII)

in which $R_2$ and $R_3$ are as defined in general formula (I) according to Claim 1, is reacted with the compound of general formula (II)

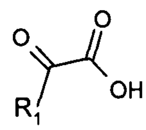

(II)

in which $R_1$ is as defined in general formula (I).

**11.** Medicinal product, **characterized in that** it contains a compound of formula (I) according to any one of Claims 1 to 8, or a salt of addition of said compound to a pharmaceutically acceptable acid, or alternatively a hydrate or a solvate of the compound of formula (I).

**12.** Pharmaceutical composition, **characterized in that** it contains a compound of formula (I) according to any one of Claims 1 to 8, or a pharmaceutically acceptable salt, a hydrate or a solvate of said compound, as well as at least one pharmaceutically acceptable excipient.

**13.** Compound of formula (I) according to any one of Claims 1 to 8, for the preparation of a medicinal product intended for the treatment, prevention, diagnosis and/or monitoring of the progress of psychiatric or neurological disorders or diseases of the central nervous system involving changes in cognitive functions, attention, ability to concentrate, capacity for learning and remembering, or the processing of sensory information, or of diseases involving spontaneous or post-traumatic neurodegenerative processes.

**14.** Compound of formula (I) according to any one of Claims 1 to 8, for the preparation of a medicinal product intended for the treatment, prevention, diagnosis and/or monitoring of the progress of acute or chronic pain.

**15.** Compound of formula (I) according to any one of Claims 1 to 8, for the preparation of a medicinal product intended for the treatment, prevention, diagnosis and/or monitoring of the progress of diseases involving inflammatory processes.

**Patentansprüche**

**1.** Verbindung der Formel (I)

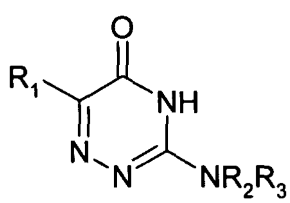

(I)

wobei:

$R_1$ für eine Heteroaryl- oder Arylgruppe steht, wobei die Heteroaryl- oder Arylgruppe gegebenenfalls durch eine oder mehrere Gruppen substituiert sind, die aus den Halogenatomen und den $(C_1-C_4)$-Alkyl-, $(C_1-C_4)$-Alkoxy-, $(C_3-C_7)$-Cycloalkyl-, Aryl-, Hydroxy-, Cyano-, $NH_2$-, $NO_2$-Gruppen ausgewählt sind;
$R_2$ für ein Wasserstoffatom oder eine $(C_1-C_4)$-Alkylgruppe steht;

$R_3$ für

- eine Gruppe $-(CH_2)n-NR_4R_5$, in der
n gleich 2, 3 oder 4 ist,
$R_4$ und $R_5$ unabhängig voneinander für eine $(C_1-C_4)$-Alkylgruppe stehen, oder R4 und R5 zusammen mit dem Stickstoffatom, das sie trägt, eine $(C_3-C_7)$-Heterocycloalkylgruppe bilden; oder
- für eine Gruppe $-(CH_2)mR_6$ steht, in der
m gleich 0, 1, 2, 3 oder 4 ist,
$R_6$ für eine $(C_3-C_9)$-Heterocycloalkylgruppe steht, die mindestens ein Stickstoffatom enthält und durch ein Kohlenstoffatom an den Triazinkern gebunden ist, wobei die $(C_3-C_9)$-Heterocycloalkylgruppe gegebenenfalls durch eine oder mehrere $(C_1-C_4)$-Alkylgruppen substituiert ist;

oder $R_2$ und $R_3$ zusammen mit dem Stickstoffatom, das sie trägt, ein $(C_5-C_9)$-Heterocycloalkyl mit 2 Stickstoffatomen bilden;
unter der Bedingung, dass wenn $R_1$ für ein Phenyl steht, $R_4$ und $R_5$ nicht gleichzeitig für ein Methyl stehen;
im basischen Zustand oder als Säureadditionssalz, sowie im Hydrat- oder Solvatzustand.

**2.** Verbindung der Formel (I) nach Anspruch 1, **dadurch gekennzeichnet, dass** $R_1$ für eine Heteroarylgruppe steht, wobei diese Heteroarylgruppe gegebenenfalls durch eine oder mehrere Gruppen substituiert ist, die aus den Halogenatomen und den $(C_1-C_4)$-Alkyl-, $(C_1-C_4)$-Alkoxy-, $(C_3-C_7)$-Cycloalkyl-, Aryl-, Hydroxy-, Cyano-, $NH_2$-, $NO_2$-Gruppen ausgewählt sind;
im basischen Zustand oder als Säureadditionssalz, sowie im Hydrat- oder Solvatzustand.

**3.** Verbindung der Formel (I) nach Anspruch 1, **dadurch gekennzeichnet, dass** $R_1$ für eine Heteroaryl- oder Arylgruppe steht, wobei diese Heteroaryl- oder Arylgruppen gegebenenfalls durch eine oder mehrere Gruppen substituiert sind, die aus den Halogenatomen und den $(C_1-C_4)$-Alkyl-, $(C_1-C_4)$-Alkoxy- und Arylgruppen ausgewählt sind;
im basischen Zustand oder als Säureadditionssalz, sowie im Hydrat- oder Solvatzustand.

**4.** Verbindung der Formel (I) nach Anspruch 1, **dadurch gekennzeichnet, dass** $R_1$ für eine Heteroarylgruppe steht, wobei diese Heteroarylgruppe gegebenenfalls durch eine oder mehrere Gruppen substituiert ist, die aus den Halogenatomen und den $(C_1-C_4)$-Alkyl-, $(C_1-C_4)$-Alkoxy- und Arylgruppen ausgewählt sind;
im basischen Zustand oder als Säureadditionssalz, sowie im Hydrat- oder Solvatzustand.

**5.** Verbindung der Formel (I) nach einem der Ansprüche 1 bis 4, **dadurch gekennzeichnet, dass** $R_2$ für ein Wasserstoffatom steht;
im basischen Zustand oder als Säureadditionssalz, sowie im Hydrat- oder Solvatzustand.

**6.** Verbindung der Formel (I) nach einem der Ansprüche 1 bis 5, **dadurch gekennzeichnet, dass**
$R_3$ für

- eine Gruppe $-(CH_2)n-NR_4R_5$, in der
n gleich 2, 3 oder 4 ist,
$R_4$ und $R_5$ unabhängig voneinander für eine $(C_1-C_4)$ - Alkylgruppe stehen, oder $R_4$ und $R_5$ zusammen mit dem Stickstoffatom, das sie trägt, eine $(C_3-C_7)$-Heterocycloalkylgruppe bilden; oder
- für eine Gruppe $-(CH_2)mR_6$ steht, in der m gleich 0, 1, 2, 3 oder 4 ist,
$R_6$ für eine $(C_3-C_9)$-Heterocycloalkylgruppe steht, die mindestens ein Stickstoffatom enthält und durch ein Kohlenstoffatom an den Triazinkern gebunden ist, wobei die $(C_3-C_9)$-Heterocycloalkylgruppe gegebenenfalls durch eine oder mehrere $(C_1-C_4)$-Alkylgruppen substituiert ist;

unter der Bedingung, dass wenn $R_1$ für ein Phenyl steht, $R_4$ und $R_5$ nicht gleichzeitig für ein Methyl stehen;
im basischen Zustand oder als Säureadditionssalz, sowie im Hydrat- oder Solvatzustand.

**7.** Verbindung der Formel (I) nach einem der Ansprüche 1 bis 6, **dadurch gekennzeichnet, dass**

$R_1$ für eine Indolylgruppe steht, wobei die Indolylgruppe gegebenenfalls durch eine oder mehrere Gruppen substituiert ist, die aus den Halogenatomen und den $(C_1-C_4)$-Alkyl-, $(C_1-C_4)$-Alkoxy-, $(C_3-C_7)$-Cycloalkyl-, Aryl-, $-NH_2$-, $-NO_2$-Gruppen ausgewählt sind;
$R_2$ für ein Wasserstoffatom steht;

$R_3$ für

- eine Gruppe $-(CH_2)n-NR_4R_5$, in der
n gleich 2, 3 oder 4 ist,
$R_4$ und $R_5$ unabhängig voneinander für eine $(C_1-C_4)$-Alkylgruppe stehen, oder $R_4$ und $R_5$ zusammen mit dem Stickstoffatom, das sie trägt, eine $(C_3-C_7)$ Heterocycloalkylgruppe bilden; oder
- für eine Gruppe $-(CH_2)mR_6$ steht, in der
m gleich 0, 1, 2, 3 oder 4 ist,

$R_6$ für eine $(C_3-C_7)$-Heterocycloalkylgruppe steht, wobei die $(C_3-C_7)$-Heterocycloalkylgruppe gegebenenfalls durch eine oder mehrere $(C_1-C_4)$-Alkylgruppen substituiert ist; im basischen Zustand oder als Säureadditionssalz, sowie im Hydrat- oder Solvatzustand.

**8.** Verbindung der Formel (I) nach einem der Ansprüche 1 bis 7, **dadurch gekennzeichnet, dass** sie ausgewählt wird aus:

- 6-(1-Methyl-1*H*-indol-3-yl)-3-(3-piperidin-1-yl-propylamino)-4*H*-1,2,4-triazin-5-on
- 3-(3-Diethylaminopropylamino)-6-(1-methyl-1*H*-indol-3-yl)-4*H*-1,2,4-triazin-5-on
- 3-(3-Diethylaminopropylamino)-6-phenyl-4*H*-1,2,4-triazin-5-on
- 3-(2-Diethylaminoethylamino)-6-(1-methyl-1*H*-indol-3-yl)-4*H*-1,2,4-triazin-5-on
- 3-(3-Dimethylaminopropylamino)-6-(1-methyl-1*H*-indol-3-yl)-4*H*-1,2,4-triazin-5-on
- 6-(1-Methyl-1*H*-indol-3-yl)-3-(3-pyrrolidin-1-ylpropylamino)-4*H*-1,2,4-triazin-5-on
- 6-(1-Methyl-1*H*-indol-3-yl)-3-[2-(1-methylpyrrolidin-2-yl)ethylamino]-4*H*-1,2,4-triazin-5-on
- 6-(1*H*-Indol-3-yl)-3-(3-pyrrolidin-1-ylpropylamino)-4*H*-1,2,4-triazin-5-on
- 6-(1*H*-Indol-3-yl)-3-(3-piperidin-1-ylpropylamino)-4*H*-1,2,4-triazin-5-on
- 3-[(*S*)-(1-Azabicyclo[2.2.2]oct-3-yl)amino]-6-(1*H*-indol-3-yl)-4*H*-1,2,4-triazin-5-on
- 3-[(*R*)-(1-Azabicyclo[2.2.2]oct-3-yl)amino]-6-(1*H*-indol-3-yl)-4*H*-1,2,4-triazin-5-on)
- 6-(Furan-3-yl)-3-(3-diethylaminopropylamino)-4*H*-1,2,4-triazin-5-on)
- 6-(Naphth-1-yl)-3-(3-diethylaminopropylamino)-4*H*-1,2,4-triazin-5-on
- 6-(1-Methyl-5-methoxyindol-3-yl)-3-(3-diethylaminopropylamino)-4*H*-1,2,4-triazin-5-on
- 6-(1-Phenylindol-3-yl)-3-(3-diethylaminopropylamino)-4*H*-1,2,4-triazin-5-on
- 6-(Indol-3-yl)-3-(3-diethylaminopropylamino)-4*H*-1,2,4-triazin-5-on
- 6-(Benzothiophen-3-yl)-3-(3-diethylaminopropylamino)-4*H*-1,2,4-triazin-5-on
- 6-(4-Methoxyphenyl)-3-(3-diethylaminopropylamino)-4*H*-1,2,4-triazin-5-on
- 6-(1-Methyl-5-fluorindol-3-yl)-3-(3-diethylaminopropylamino)-4*H*-1,2,4-triazin-5-on
- 6-(1-Methyl-6-fluorindol-3-yl)-3-(3-diethylaminopropylamino)-4*H*-1,2,4-triazin-5-on
- 6-(3-Methoxyphenyl)-3-(3-diethylaminopropylamino)-4*H*-1,2,4-triazin-5-on
- 6-(4-(4-Methylphenyl))-3-(3-diethylaminopropylamino)-4*H*-1,2,4-triazin-5-on
- 6-(1-Methyl-4-fluorindol-3-yl)-3-(3-diethylaminopropylamino)-4*H*-1,2,4-triazin-5-on.

**9.** Verfahren zur Herstellung einer Verbindung der Formel (I) nach einem der Ansprüche 1 bis 8, **dadurch gekennzeichnet, dass** die Verbindung der allgemeinen Formel (V)

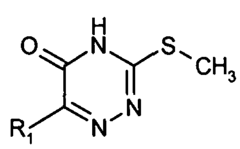

(V)

in der $R_1$ wie in der allgemeinen Formel (I) nach Anspruch 1 definiert ist,
mit einem Amin der allgemeinen Formel $HNR_2R_3$ (VI), in der $R_2$ und $R_3$ wie in der allgemeinen Formel (I) nach Anspruch 1 definiert sind, zur Reaktion gebracht wird.

**10.** Verfahren zur Herstellung einer Verbindung der Formel (I) nach einem der Ansprüche 1 bis 8, **dadurch gekennzeichnet, dass** die Verbindung der allgemeinen Formel (VII),

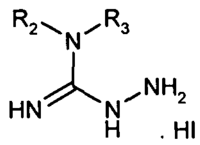

(VII)

in der $R_2$ und $R_3$ wie in der allgemeinen Formel (I) nach Anspruch 1 definiert sind, mit der Verbindung der allgemeinen Formel (II)

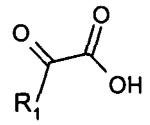

(II)

in der $R_1$ wie in der allgemeinen Formel (I) definiert ist, zur Reaktion gebracht wird.

**11.** Arzneimittel, **dadurch gekennzeichnet, dass** es eine Verbindung der Formel (I) nach einem der vorhergehenden Ansprüche 1 bis 8 oder ein Additionssalz dieser Verbindung mit einer pharmazeutisch unbedenklichen Säure oder auch ein Hydrat oder ein Solvat der Verbindung der Formel (I) umfasst.

**12.** Pharmazeutische Zusammensetzung, **dadurch gekennzeichnet, dass** sie eine Verbindung der Formel (I) nach einem der vorhergehenden Ansprüche 1 bis 8 oder ein pharmazeutisch unbedenkliches Salz, ein Hydrat oder ein Solvat dieser Verbindung sowie mindestens ein pharmazeutisch unbedenkliches Hilfsmittel umfasst.

**13.** Verbindung der Formel (I) nach einem der Ansprüche 1 bis 8 zur Herstellung eines Arzneimittels, das zur Behandlung, Vorbeugung, Diagnose und/oder zum Verfolgen der Entwicklung von psychiatrischen oder neurologischen Störungen oder Krankheiten des zentralen Nervensystems, einschließlich der Veränderungen der kognitiven Funktionen, der Aufmerksamkeit, der Konzentrationsfähigkeiten, der Fähigkeiten zum Lernen und zur Wissensspeicherung oder der Behandlung sensorieller Informationen, sowie von Krankheiten bestimmt ist, die spontane oder als Folge von Verletzungen auftretende, neurodegenerative Prozesse umfassen.

**14.** Verbindung der Formel (I) nach einem der Ansprüche 1 bis 8 zur Herstellung eines Arzneimittels, das zur Behandlung, Vorbeugung, Diagnose und/oder zum Verfolgen der Entwicklung von akuten oder chronischen Schmerzen bestimmt ist.

**15.** Verbindung der Formel (I) nach einem der Ansprüche 1 bis 8 zur Herstellung eines Arzneimittels, das zur Behandlung, Vorbeugung, Diagnose und/oder zum Verfolgen der Entwicklung von Krankheiten bestimmt ist, die entzündliche Phänomene umfassen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 2002030084 B
- WO 03029252 A1
- WO 2003018585 A1
- WO 2002085901 A1
- WO 2003029252 A1
- WO 2002068420 A1
- EP 115933 A2
- WO 2003070728 A2


### Littérature non-brevet citée dans la description


- **Ried W.** *Liebigs annalen der chemie,* 1988, 141-8
- **Lavergne, J.-P.** *Bulletin de la Société Chimique de France,* 1976, 1827-8
- **Lalezari, I.** *Journal of Heterocyclic Chemistry,* 1976, vol. 13, 1249-51
- **Burch, H.** *Journal of Medicinal Chemistry,* 1970, vol. 13 (2), 288-91
- *Tetrahedron Lett.,* 1994, vol. 35 (19), 3013-3016
- *Tetrahedron Lett.,* 1998, vol. 39 (52), 9629-9630
- *J. Org. Chem.,* 2002, vol. 67 (17), 6226-6227
- *Synthesis,* 1998, 1241-1242
- *J. Heterocycl. Chem.,* 1997, vol. 34 (2), 441-444
- *J. Heterocycl. Chem.,* 1997, vol. 34 (3), 789-795
- *Tetrahedron,* 2003, vol. 59 (7), 1083-1094
- *Bioorg. Med. Chem. Lett.,* 2002, vol. 12 (7), 1117-1120
- *Heterocycles,* 1998, vol. 49 (1), 459-464
- *J. Org. Chem.,* 1981, vol. 46 (1), 211-213
- *Tetrahedron,* vol. 52 (42), 13513-13520
- *Tetrahedron,* 1999, vol. 55 (37), 11343-11364
- *J. Org. Chem.,* 1987, vol. 52 (26), 5733-5740
- *Adv. Synth. Catal.,* 2001, vol. 343 (3), 289-298
- *Tetrahedron Lett.,* 1983, vol. 24 (33), 3455-3456
- *Synth. Comm.,* 2002, vol. 32 (13), 1985-1995
- *J. Med. Chem.,* 1993, vol. 36 (6), 683-689
- *Tetrahedron,* 1992, vol. 49 (2), 451-468
- *Hev. Chim. Acta,* 1955, vol. 38 (3), 559-570
- *Hev. Chim. Acta,* 1959, vol. 42 (1), 67-72
- *J. Med. Chem.,* 1993, vol. 36 (16), 2311-20
- *J. Org. Chem.,* 1996, vol. 61 (11), 3766-3772
- **Davies et al.** *Neuropharmacology,* 1999, vol. 38, 679-690
- **Cheng ; Prusoff.** *Biochem. Pharmacol.,* 1973, vol. 22, 3099-3108